# EUROPÄISCHE PATENTSCHRIFT

(11) **EP 0 842 144 B1**
(45) Veröffentlichungstag und Bekanntmachung des Hinweises auf die Patenterteilung: **31.10.2001**
(21) Anmeldenummer: 96925734.4
(22) Anmeldetag: 12.07.1996
(51) Int. Cl.: C07C 251/60, C07C 255/64, C07C 331/12, C07D 231/20, C07C 329/06, C07C 251/34, C07C 251/50, A01N 37/36, A01N 37/50, A01N 47/48, A01N 43/56

(54) **PHENYLESSIGSÄUREDERIVATE, VERFAHREN UND ZWISCHENPRODUKTE ZU IHRER HERSTELLUNG UND IHRE VERWENDUNG ALS SCHÄDLINGSBEKÄMPFUNGSMITTEL UND FUNGIZIDE**
PHENYL ACETIC ACID DERIVATIVES, PROCESS AND INTERMEDIATE PRODUCTS FOR THEIR PRODUCTION AND THEIR USE AS PARASITICIDES AND FUNGICIDES
DERIVES D'ACIDE ACETIQUE PHENYLIQUE, PROCEDES ET PRODUITS INTERMEDIAIRES PERMETTANT DE LES PRODUIRE ET UTILISATION COMME AGENTS DE LUTTE CONTRE LES PARASITES ET COMME FONGICIDES

(30) Priorität: 27.07.1995 DE 19527466; 07.11.1995 DE 19541382
(43) Veröffentlichungstag der Anmeldung: 20.05.1998
(73) Patentinhaber: BASF AKTIENGESELLSCHAFT, 67056 Ludwigshafen (DE)
(72) Erfinder: MÜLLER, Ruth, D-67159 Friedelsheim (DE); BAYER, Herbert, D-68159 Mannheim (DE); SAUTER, Hubert, D-68167 Mannheim (DE); HARREUS, Albrecht, D-67063 Ludwigshafen (DE); MÜLLER, Bernd, D-67227 Frankenthal (DE); STRATHMANN, Siegfried, D-67117 Limburgerhof (DE); LORENZ, Gisela, D-67434 Hambach (DE); AMMERMANN, Eberhard, D-64646 Heppenheim (DE); HARRIES, Volker, D-67227 Frankenthal (DE); GÖTZ, Norbert, D-67547 Worms (DE); GROTE, Thomas, D-67105 Schifferstadt (DE)
(86) Internationale Anmeldenummer: EP9603057
(87) Internationale Veröffentlichungsnummer: WO9705103

(56) Entgegenhaltungen:
- EP-A- 0 414 153
- WO-A-95/18789
- WO-A-95/21153
- WO-A-95/21154
- CHEMICAL ABSTRACTS, vol. 51, no. 16, 25.August 1957 Columbus, Ohio, US; abstract no. 11854b, XP002018967 & ANN. CHIM., Bd. 46, 1956, Seiten 571-81, E. BORELLO ET AL.: "Ultraviolet spectra and structure of the glyoximes"
- CHEMICAL ABSTRACTS, vol. 119, no. 5, 2.August 1993 Columbus, Ohio, US; abstract no. 44353, SCHWOCHAU, K. ET AL: "Preparation, characterization and biological evaluation of cationic 99Tc/99mTc-dioxime complexes" XP002018968 & NUCL. MED. BIOL. (1993), 20(3), 317-24,
- TETRAHEDRON (1992), 48(21), 4407-418, XP002018966 DELL'ERBA, CARLO ET AL: "Synthetic exploitation of the ring-opening of 3,4-dinitrothiophene. Access to 1,4-disubstituted 2,3-dinitro-1,3-butadienes and 2,3-butanedione dioximes"
- CHEMICAL ABSTRACTS, vol. 97, no. 7, 16.August 1982 Columbus, Ohio, US; abstract no. 55275, NIKOLAEVA, A. D. ET AL: "Synthesis of.alpha.-(hydroxyimino).beta.-chloro ketones" XP002018969 & DEPOSITED DOC. (1980), SPSTL 613KHP-D80, 8 PP. AVAIL.: SPSTL,
- CHEMICAL ABSTRACTS, vol. 66, no. 12, 20.März 1967 Columbus, Ohio, US; abstract no. 51793, UHLIG, EGON ET AL: "Oxime complexes. V. Nickel(II) and palladium(II) chelates of amino derivatives of dimethylglyoxime" XP002018970 & Z. ANORG. ALLG. CHEM. (1966), 348(1-2), 12-20,
- CHEMICAL ABSTRACTS, vol. 63, no. 5, 30.August 1965 Columbus, Ohio, US; abstract no. 5599d, G. BUCHMANN ET AL.: "Chemistry of 8-quinolyl mercaptan" XP002018971 & J. PRAKT. CHEM., Bd. 28, Nr. 3-4, 1965, Seiten 141-56,

## Beschreibung

Die vorliegende Erfindung betrifft Phenylessigsäurederivate der Formel I 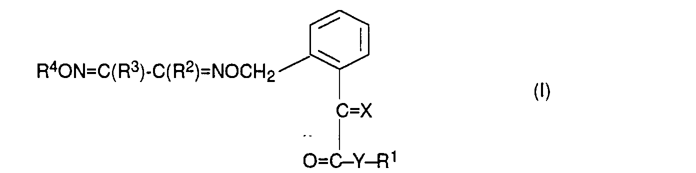 in der die Substituenten und der Index die folgende Bedeutung haben:
- X: NOCH₃, CHOCH₃ und CHCH₃;
- Y: NR und O;
- R, R¹: Wasserstoff und C₁-C₄-Alkyl;
- R²: Methyl;
- R³: C₂-C₆-Alkenyl;
- R⁴: Wasserstoff, C₁-C₆-Alkyl, C₂-C₆-Alkenyl, C₂-C₆-Alkinyl, wobei die Reste partiell oder vollständig halogeniert sein können und/oder 1 - 3 der folgenden Substituenten tragen können: Cyano, Hydroxy, Amino, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, Aminocarbonyl, C₁-C₆-Alkylaminocarbonyl, Di-C₁-C₆-alkylaminocarbonyl, Alkenyloxy, C₃-C₆-Cycloalkyl, C₃-C₆-Cycloalkyloxy, wobei die cyclischen Gruppen ihrerseits partiell oder vollständig halogeniert sein können oder einen bis drei der Reste C₁-C₃-Alkyl oder C₁-C₃-Alkoxy tragen können,
sowie deren Salze.

Außerdem betrifft die Erfindung Verfahren und Zwischenprodukte zur Herstellung dieser Verbindungen sowie sie enthaltende Mittel zur Bekämpfung tierischer Schädlinge und Schadpilze.

Aus der Literatur sind Phenylessigsäurederivate zur Schädlingsbekämpfung bekannt [EP-A 422 597, EP-A 463 488, EP-A 370 629, EP-A 460 575, EP-A 472 300, WO-A 90/07,493, WO-A 92/13,830, WO-A 92/18,487; WO-A 95/18,789; WO-A 95/21,153; WO-A 95/21,154].

Der vorliegenden Erfindung lagen neue Verbindungen mit verbesserter Wirkung als Aufgabe zugrunde.

Demgemäß wurden die eingangs definierten Phenylessigsäurederivate I gefunden. Außerdem wurden Verfahren und Zwischenprodukte zu ihrer Herstellung sowie sie enthaltende Mittel zur Bekämpfung von tierischen Schädlingen und Schadpilzen und ihre Verwendung in diesem Sinne gefunden.

Die Verbindungen I sind auf verschiedenen Wegen nach an sich in der Literatur bekannten Verfahren erhältlich.

Grundsätzlich ist es bei der Synthese der Verbindungen I unerheblich, ob zunächst die Gruppierung -C(X)-COYR¹ oder die Gruppierung -CH₂ON=C(R²)-C(R³)=NOR⁴ aufgebaut wird.

Der Aufbau der Gruppierung -C(X)-COYR¹ ist beispielsweise aus der eingangs zitierten Literatur sowie aus den folgenden Literaturstellen bekannt: EP-A 242 070, EP-A 254 426, EP-A 370 629, EP-A 513 580, EP-A 656 352, PCT/EP 95/02013, EP-A 398 692.
1. Man geht beim Aufbau der Gruppierung -CH₂ON=C(R²)-C(R³)=NOR⁴ im allgemeinen so vor, daß man ein Benzylderivat der Formel II mit einem Hydroxyimin der Formel III umsetzt. 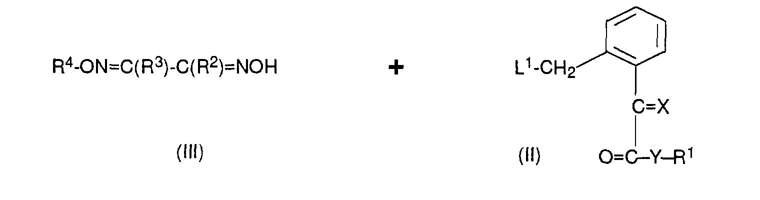 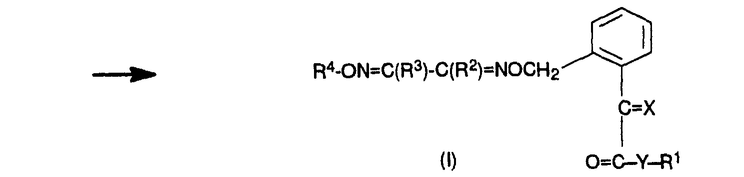 L¹ in der Formel II steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Natriumhydrid, Kaliumhydroxid, Kaliumcarbonat und Triethylamin gemäß den in Houben-Weyl, Bd. E 14b, S. 370f und Houben-Weyl, Bd. 10/1, S. 1189f beschriebenen Methoden.
   Das benötigte Hydroxyimin III erhält man beispielsweise durch Umsetzung eines entsprechenden Dihydroxyimins IV mit einem nukleophil substituierten Reagens VI 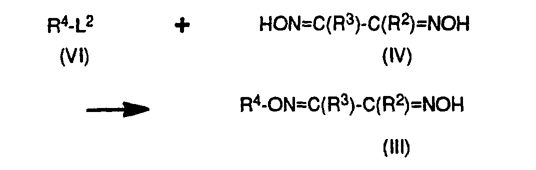 L² in der Formel VI steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Kaliumcarbonat, Kaliumhydroxid, Natriumhydrid, Pyridin und Triethylamin gemäß den in Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f, beschriebenen Methoden.
   1.1 Alternativ können die Verbindungen I auch dadurch erhalten werden, daß das Benzylderivat II zunächst mit dem Dihydroxyiminoderivat IV in ein entsprechendes Benzyloxim der Formel V umgesetzt wird, wobei V anschließend mit dem nukleophil substituierten Reagens VI zu I umgesetzt wird. 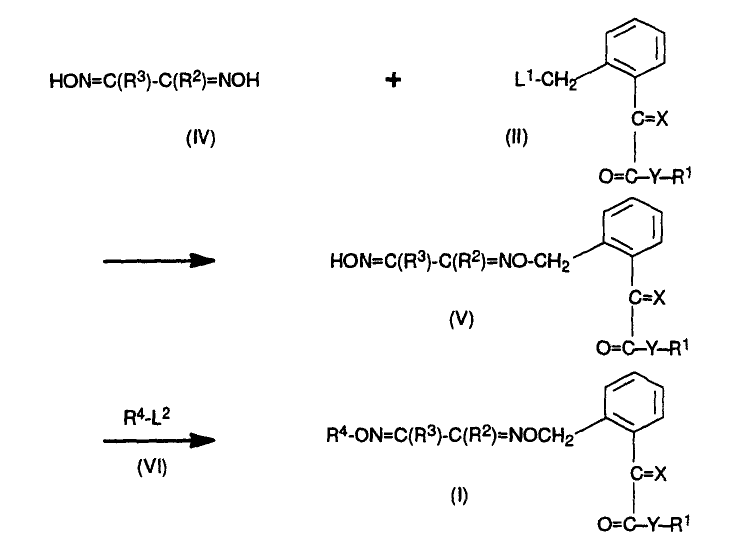
      Die Umsetzung erfolgt in an sich bekannter Weise in einem inerten organischen Lösungsmittel in Gegenwart einer Base z.B. Kaliumcarbonat, Kaliumhydroxid, Natriumhydrid, Pyridin und Triethylamin gemäß den in Houben-Weyl, Bd. 10/1, S. 1189f; Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f, beschriebenen Methoden.
   1.2 Analog ist es ebenfalls möglich, das benötigte Hydroxyimin der Formel III aus einem Carbonylhydroxyimin VII durch Umsetzung mit einem Hydroxylamin IXa oder seinem Salz IXb herzustellen. 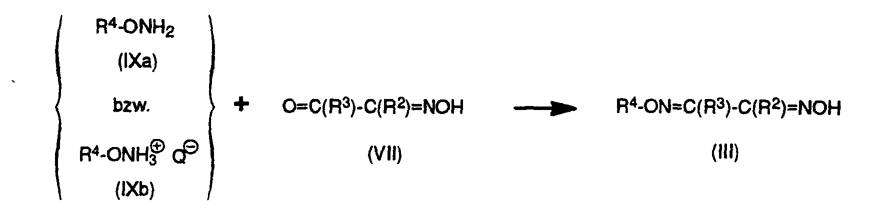 Q^{⊖} in der Formel IXb steht für das Anion einer Säure, insbesondere einer anorganischen Säure, z.B. Halogenid wie Chlorid.
      Die Umsetzung erfolgt in an sich bekannter Weise in einem inerten organischen Lösungsmittel gemäß den in EP-A 513 580; Houben-Weyl, Bd. 10/4, S. 73f; Houben-Weyl, Bd. E 14b, S. 369f und S. 385f beschriebenen Methoden.
      Die Carbonylhydroxyimine VII sind entweder literaturbekannt (z.B. Chem. Ber. 46 (1913) 1864; J. Chem. Soc. Perkin Trans. 2 (1994) 3, 415-420; Gazz. Chim. Ital. 63 (1933) 917 f.; J. Indian. Chem. Soc. 35 (1958) 898) oder können nach analogen Verfahren hergestellt werden.
   1.3 Alternativ können die Verbindungen I auch dadurch erhalten werden, daß das Benzylderivat II zunächst mit dem Carbonylhydroxyiminoderivat VII in ein entsprechendes Benzyloxyimin der Formel VIII umgesetzt wird, wobei VIII anschließend mit dem Hydroxylamin IXa bzw. dessen Salz IXb zu I umgesetzt wird. 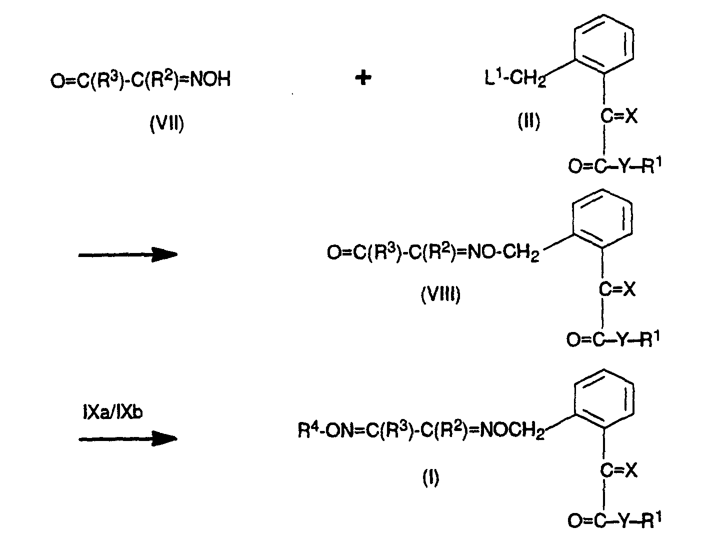
      Die Umsetzung erfolgt in an sich bekannter Weise in einem inerten organischen Lösungsmittel gemäß den in Houben-Weyl, Bd. E 14b, S. 369f; Houben-Weyl, Bd. 10/1, S. 1189f und Houben-Weyl, Bd. 10/4, S. 73f oder EP-A 513 580 beschriebenen Methoden.
   1.4 Eine weitere Möglichkeit zur Herstellung der Verbindungen I ist die Umsetzung des Benzylderivats II mit N-Hydroxyphthalimid und nachfolgender Hydrazinolyse zum Benzylhydroxylamin IIa und die weitere Umsetzung von IIa mit einer Carbonylverbindung X. 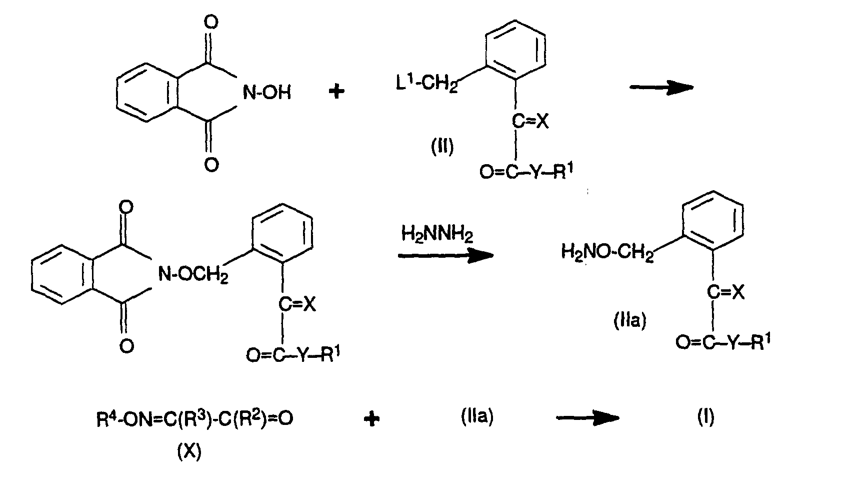
      Die Umsetzung erfolgt in an sich bekannter Weise in einem inerten organischen Lösungsmittel gemäß den in EP-A 463 488, DE-Anm. Nr. 42 28 867.3 beschriebenen Methoden.
      Die benötigte Carbonylverbindung X erhält man beispielsweise durch Umsetzung einer entsprechenden Hydroxyiminocarbonylverbindung VIIa mit einem nukleophil substituierten Reagens VI 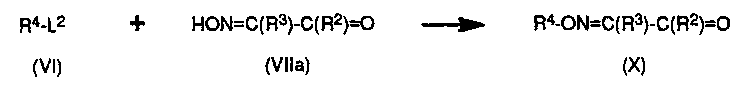 oder durch Umsetzung einer entsprechenden Dicarbonylverbindung XI mit einem Hydroxylamin IXa oder dessen Salz IXb 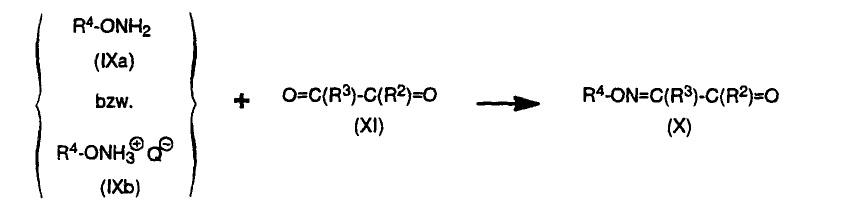
      Die Umsetzungen erfolgen in an sich bekannter Weise in einem inerten organischen Lösungsmittel gemäß den in EP-A 513 580, Houben-Weyl, Bd. 10/4, S. 55f, S. 73f, S. 180f und S. 217f; Houben-Weyl, Bd. E 14b, S. 307f und 369f, Houben-Weyl, Bd. E 5, S. 780f beschriebenen Methoden.
   1.5 Entsprechend können die Verbindungen I auch dadurch erhalten werden, daß das Benzylhydroxylamin IIa zunächst mit dem Hydroxyiminoderivat VIIa in das entsprechende Benzyloxyiminoderivat der Formel V umgesetzt wird, wobei V anschließend mit dem nukleophil substituierten Reagens VI wie vorstehend beschrieben zu I umgesetzt wird. 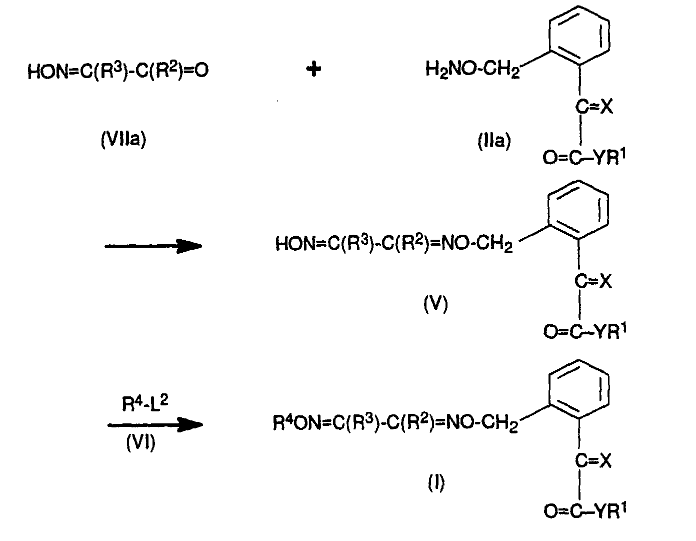
   1.6 Analog können die Verbindungen I ebenfalls dadurch hergestellt werden, daß das Benzylhydroxylamin IIa zunächst mit dem Dicarbonylderivat der Formel XI in das Benzyloxyiminoderivat der Formel VIII überführt wird und VIII anschließend mit dem Hydroxylamin IXa oder dessen Salz IXb wie vorstehend beschrieben zu I umgesetzt wird. 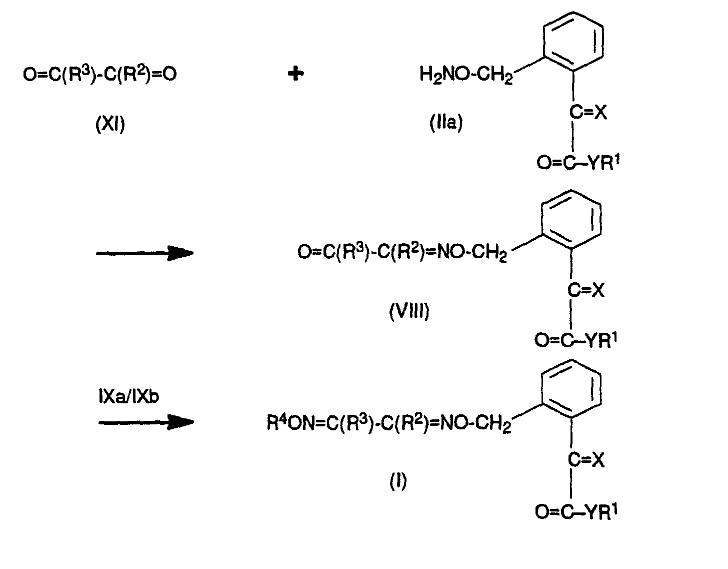
   1.7 Verbindungen VIII können ebenfalls ausgehend von Verbindung VIIIa erhalten werden.
      Hierbei wird die C=C-Doppelbindung des Alkenylrestes durch Umsetzung von VIIIa mit einer Carbonylverbindung VIIIb in an sich bekannter Weise im Sinne einer Aldolreaktion aufgebaut (z.B. Organikum, 15. Auflage, S. 563 ff.), wobei die Substituenten R³', R³'' und R³''' über den Rest R³ vorgegeben sind: 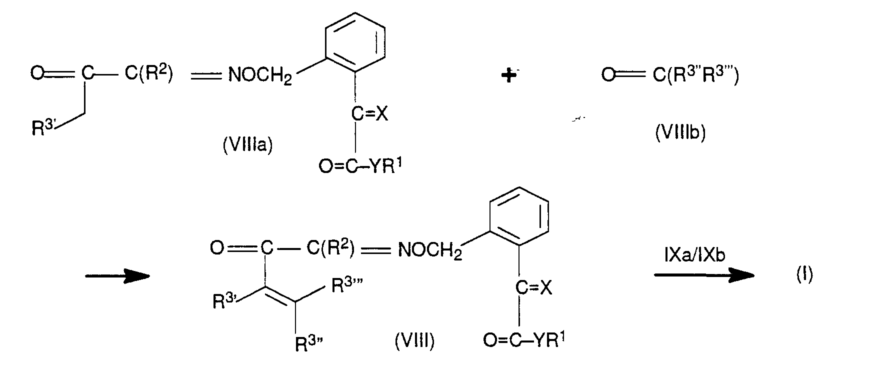 Verbindung VIII können wie vorstehend beschrieben, zu Verbindungen I umgesetzt werden.
   1.8 Verbindungen I können außerdem ausgehend von den Halogenverbindungen VIIIc gemäß dem folgenden Reaktionsschema synthetisiert werden. Zur besseren Übersichtlichkeit ist in diesem Reaktionsschema die Gruppe C(=X)-COYR¹ verkürzt als # dargestellt. 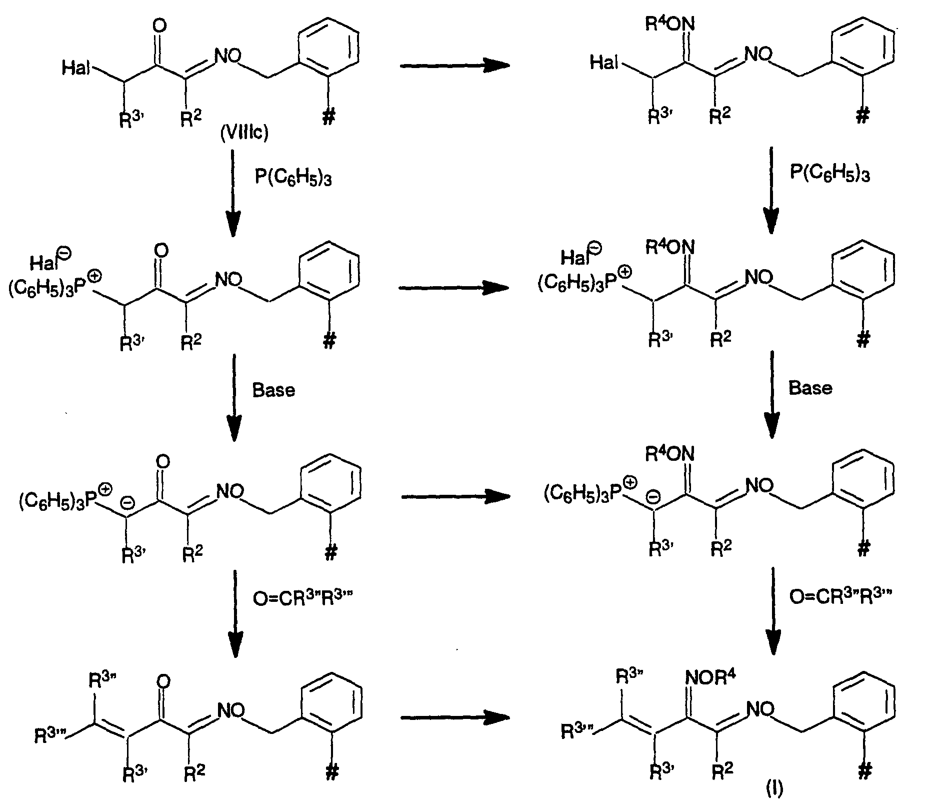
      Hierbei wird die C=C-Doppelbindung des Alkenylrestes in an sich bekannter Weise über eine Wittig-Reaktion (gemäß der schematischen Darstellung; vgl. Houben-Weyl, Bd. V1b, 4. Auflage, S. 383f) oder in entsprechender Weise über eine Wittig-Horner Reaktion aufgebaut. Die Gruppierung CR³'=CR³"R³'" entspricht dem Alkenylrest R³.
2. Verbindungen I, in denen Y Sauerstoff bedeutet, erhält man bevorzugt dadurch, daß man eine Verbindung X gemäß den in EP-A 493 711 beschriebenen Methoden mit einem Lacton XII zunächst in die entsprechende Benzoesäure XIII überführt und XIII über die entsprechenden Halogenide in die Cyanocarbonsäuren XIV überführt, welche im Wege der Pinner-Reaktion (Angew. Chem. 94, 1 (1982)) in die α-Ketoester XV und anschließend in die Derivate I überführt werden (vgl. EP-A 348 766, DE-A 37 05 389, EP-A 178 826, DE-A 36 23 921). 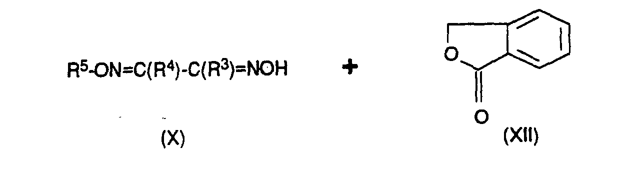 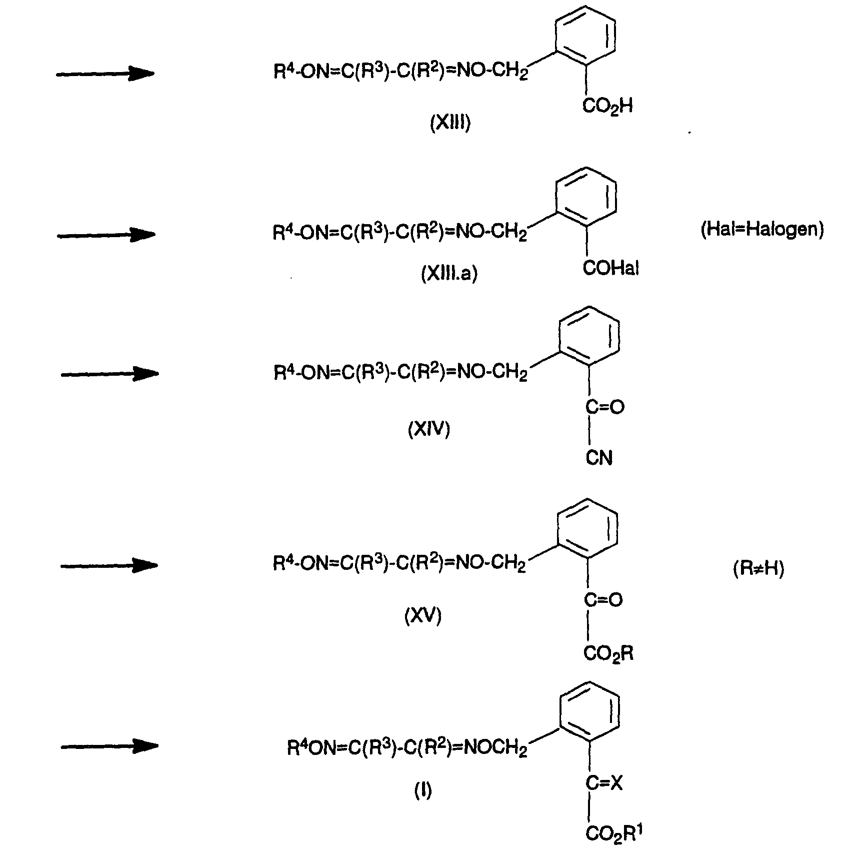 Verbindungen I, in denen R¹ Wasserstoff bedeutet, erhält man nach diesem Verfahren durch Verseifung der Ester XV und anschließende Umsetzung zu I.
3. Verbindungen I, in denen Y NR bedeutet, erhält man bevorzugt dadurch, daß man eine Verbindung X gemäß den in EP-A 493 711 beschriebenen Methoden mit einem Lacton XII zunächst in die entsprechende Benzoesäure XIII überführt und XIII über die entsprechenden Halogenide in die Cyanocarbonsäuren XIV überführt, welche dann im Wege der Pinner-Reaktion (Angew. Chem. 94, 1 (1982)) in die α-Ketoester XV überführt werden. Aus den Derivaten XV erhält man durch Amidierung die entsprechenden Carbonsäureamide XVI, die anschließend in die Verbindungen I überführt werden. 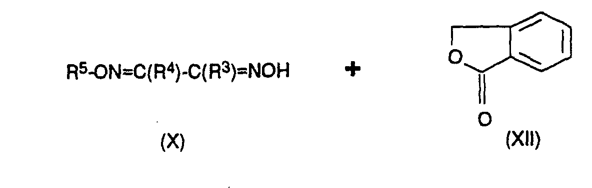 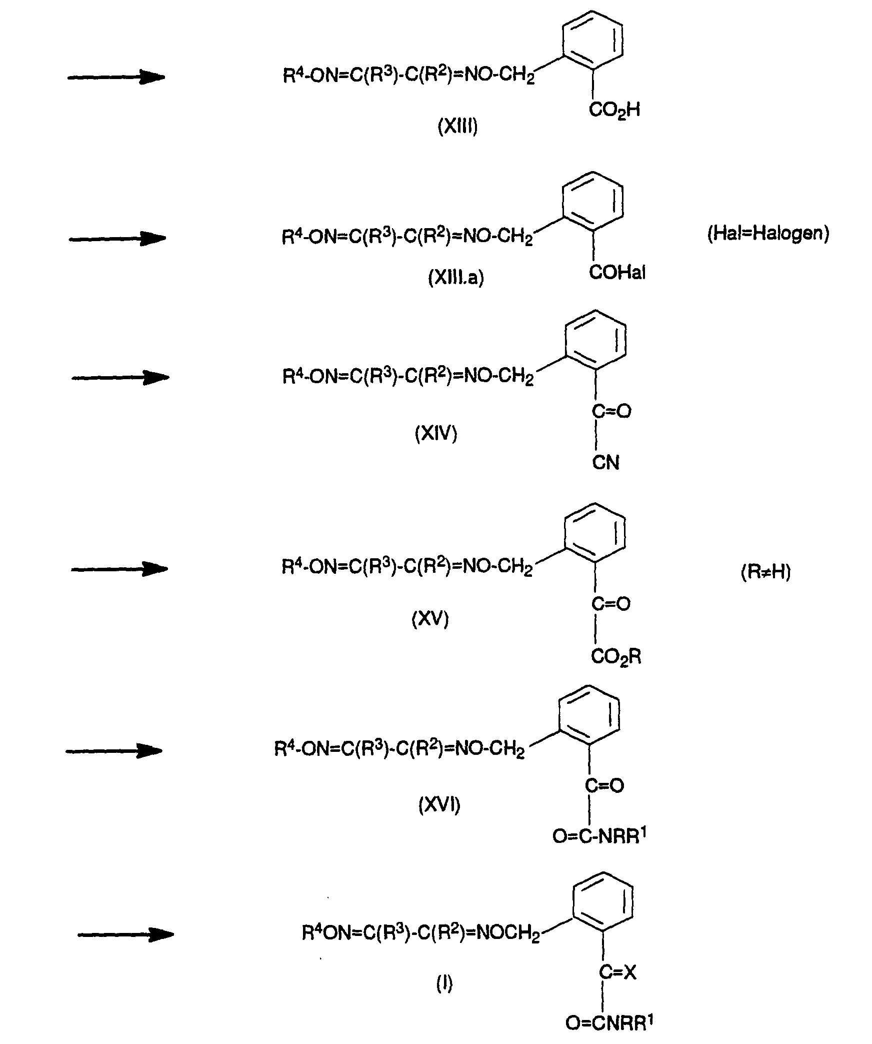
4. In Abwandlung des unter 3. beschriebenen Verfahrens kann man auch direkt aus den Carbonsäurehalogeniden durch Umsetzung mit Isocyanaten und anschließender Hydrolyse die Verbindungen XVI erhalten, in denen R Wasserstoff bedeutet (EP-A 547 825). 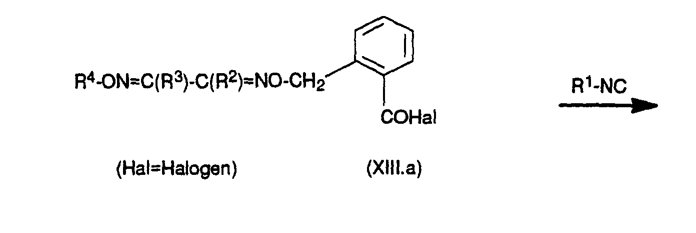 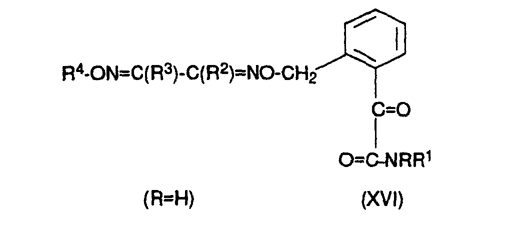
5. In einer weiteren Variante erhält man Verbindungen XVI dadurch, daß man eine ortho-Halogenverbindung nach Metallierung mit Oxalylchlorid in das entsprechende Ketosäurechlorid überführt, welches anschließend mit einem Amin in das entsprechende Amid XVI überführt wird (vgl. J. Org. Chem. 46, 212 f (1981); DE-A 40 42 280; Houben Weyl, Bd. E5, S. 972 f). 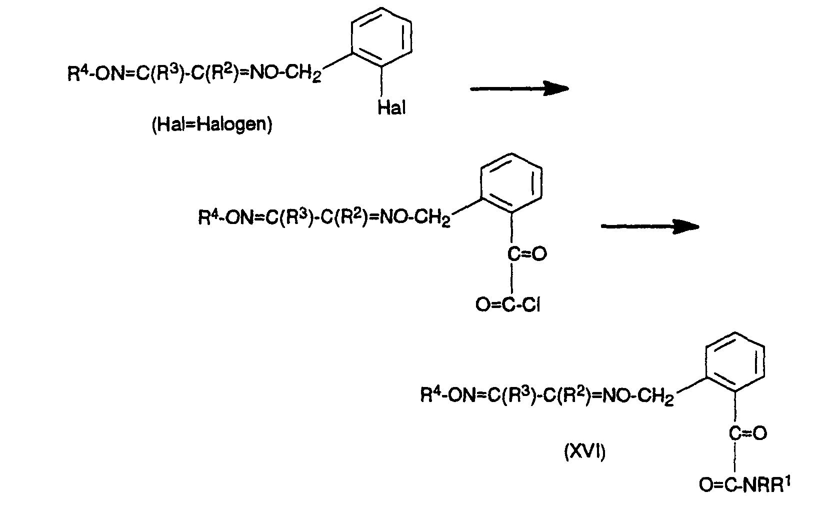
6. In einer weiteren Variante erhält man die Verbindungen I in denen Y NR bedeutet, ausgehend von den Ketoestern XV, indem man zunächst die Ketofunktion derivatisiert und die so erhaltenen Derivate mit einem entsprechenden Amin in das Amid überführt (Houben-Weyl, Bd. E5, S. 941 f.)

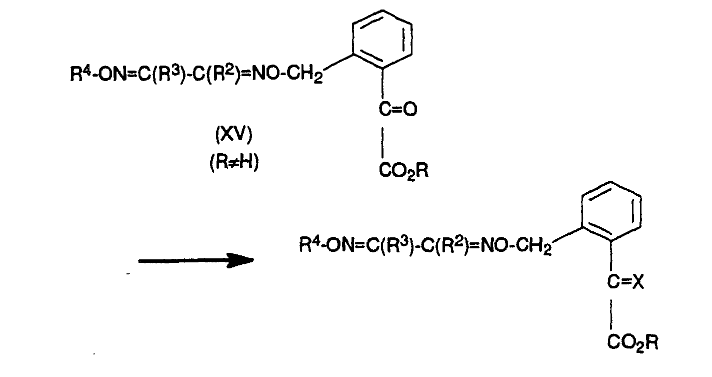

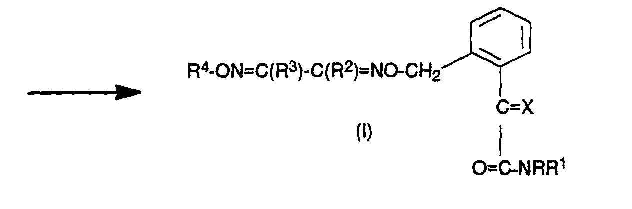

Die Verbindungen II sind bekannt (EP-A 513 580, EP-A 477 631, EP-A 463 488, EP-A 585 751, EP-A 400 417, EP-A 251 082) oder können nach den dort beschriebenen Methoden hergestellt werden.

Die Verbindungen I können bei der Herstellung aufgrund ihrer C=C und C=N Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die C=X Doppelbindung werden hinsichtlich ihrer Wirksamkeit die E-Isomere der Verbindungen I bevorzugt (Konfiguration bezogen auf die -OCH₃ bzw. die -CH₃ Gruppe im Verhältnis zur (O=C)YR¹ Gruppe).

In Bezug auf die -C(R²)=NOCH₂- Doppelbindung werden hinsichtlich ihrer Wirksamkeit im allgemeinen die cis-Isomere der Verbindungen I bevorzugt (Konfiguration bezogen auf den Rest R² im Verhältnis zur -OCH₂- Gruppe).

Bei der eingangs angegebenen Definitionen der Verbindungen I wurden Sammelbegriffe verwendet, die allgemein repräsentativ für die folgenden Gruppen stehen:

### Halogen: Fluor, Chlor, Brom und Jod;

**Alkyl:** geradkettige oder verzweigte Alkylgruppen mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 4 oder 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, l-Ethyl-l-methylpropyloxy und 1-Ethyl-2-methylpropyloxy;
**Halogenalkoxy:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, und wobei diese Gruppen über ein Sauerstoffatom an das Gerüst gebunden sind;
**Alkenyl:** geradkettige oder verzweigte Alkenylgruppen mit 2 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Eth yl-3-butenyl, 1,1,2-Trimethyl-2-propenyl, 1- Ethyl-1-methyl-2-propenyl, 1-Ethyl-2-methyl-1-propenyl und 1-Ethyl-2-methyl-2-propenyl;
**Alkinyl:** geradkettige oder verzweigte Alkinylgruppen mit 2 bis 6 Kohlenstoffatomen und einer Dreifachbindung in einer beliebigen Position, z.B. Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;

Die Angabe *"partiell oder vollständig halogeniert"* soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung sind Verbindungen der Formel I bevorzugt, in denen Y für Sauerstoff und R¹ für Methyl steht.

Außerdem bevorzugt sind Verbindungen der Formel I, in denen X NOCH₃ und Y NR oder Sauerstoff bedeutet.

Gleichermaßen bevorzugt sind Verbindungen der Formel I, in denen X NOCH₃ und Y NR bedeutet und R für Wasserstoff oder Methyl steht.

Gleichermaßen bevorzugt sind Verbindungen der Formel I, in denen X NOCH₃ und Y NR bedeutet und R¹ für Wasserstoff oder Methyl steht.

Des weiteren bevorzugt sind Verbindungen der Formel I, in denen X NOCH₃ und Y NR bedeutet und R und R¹ gleichzeitig Wasserstoff oder Methyl bedeuten.

Des weiteren bevorzugt sind Verbindungen der Formel I, in denen X NOCH₃ und Y NR bedeutet und R für Wasserstoff und R¹ für Methyl steht.

Daneben werden Verbindungen der Formel I bevorzugt, in denen X für NOCH₃ und Y für O steht.

Daneben werden Verbindungen der Formel I bevorzugt, in denen X für CHOCH₃ und Y für O steht.

Daneben werden Verbindungen der Formel I bevorzugt, in denen X für CHCH₃ und Y für O steht.

Außerdem werden Verbindungen I bevorzugt, deren Rest R³ keine oder eine Verzweigung aufweist.

Es werden gleichermaßen Verbindungen I bevorzugt, in denen R³ für Vinyl, Allyl, Prop-1-enyl, 2-Methylprop-1-enyl oder iso-Propenyl steht.

Gleichermaßen werden Verbindungen I bevorzugt, in denen R⁴ Wasserstoff bedeutet.

Des weiteren werden Verbindungen I bevorzugt, in denen R⁴ für C₁-C₆-Alkyl steht.

Außerdem werden Verbindungen I bevorzugt, in denen R⁴ für Methyl oder Ethyl steht.

Außerdem werden Verbindungen I bevorzugt, in denen R⁴ für gegebenenfalls substituiertes C₁-C₆-Alkyl steht.

Außerdem werden Verbindungen I bevorzugt, in denen R⁴ gegebenenfalls substituiertes C₂-C₆-Alkenyl bedeutet.

Des weiteren werden Verbindungen I bevorzugt, in denen R⁴ gegebenenfalls subst. C₂-C₆-Alkinyl bedeutet.

Daneben werden Verbindungen I bevorzugt, in denen R⁴ für C₁-C₆-Alkyl steht, wobei der Alkylrest partiell oder vollständig halogeniert sein kann und/oder 1 - 3 der folgenden Substituenten tragen kann: Cyano, Hydroxy, Amino, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, Aminocarbonyl, C₁-C₆-Alkylaminocarbonyl, Di-C₁-C₆-alkylaminocarbonyl, Alkenyloxy, C₃-C₆-Cycloalkyl, C₃-C₆-Cycloalkyloxy, wobei die cyclischen Gruppen ihrerseits partiell oder vollständig halogeniert sein können oder einen bis drei der Reste C₁-C₃-Alkyl oder C₁-C₃-Alkoxy tragen können.

Daneben werden Verbindungen I bevorzugt, in denen R⁴ C₂-C₆-Alkenyl bedeutet, wobei der Alkenylrest partiell oder vollst. halogeniert sein kann oder 1 - 3 der folgenden Substituenten tragen kann: Cyano, Hydroxy, Amino, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, Aminocarbonyl, C₁-C₆-Alkylaminocarbonyl, Di-C₁-C₆-alkylaminocarbonyl, Alkenyloxy, C₃-C₆-Cycloalkyl, C₃-C₆-Cycloalkyloxy, wobei die cyclischen Gruppen ihrerseits partiell oder vollständig halogeniert sein können oder einen bis drei der Reste C₁-C₃-Alkyl oder C₁-C₃-Alkoxy tragen können.

Daneben werden Verbindungen I bevorzugt, in denen R⁴ C₂-C₆-Alkinyl bedeutet, wobei der Alkinylrest partiell oder vollständig halogeniert sein kann und/oder 1 - 3 der folgenden Substituenten tragen kann: Cyano, Hydroxy, Amino, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, Aminocarbonyl, C₁-C₆-Alkylaminocarbonyl, Di-C₁-C₆-alkylaminocarbonyl, Alkenyloxy, C₃-C₆-Cycloalkyl, C₃-C₆-Cycloalkyloxy, wobei die cyclischen Gruppen ihrerseits partiell oder vollständig halogeniert sein können oder einen bis drei der Reste C₁-C₃-Alkyl oder C₁-C₃-Alkoxy tragen können.

Daneben werden Verbindungen I bevorzugt, in denen R⁴ C₂-C₆-Alkinyl bedeutet, wobei der Alkinylrest 1 - 3 der folgenden Substituenten tragen kann: Halogen, Cyano und C₁-C₃-Alkoxy, insbesondere Halogen.

Daneben werden Verbindungen I bevorzugt, in denen R⁴ C₂-C₆-Alkenyl bedeutet, wobei der Alkenylrest 1 - 3 der folgenden Reste tragen kann: Halogen, Cyano und C₁-C₃-Alkoxy, insbesondere Halogen.

Daneben werden Verbindungen I bevorzugt, in denen R⁴ C₁-C₆-Alkyl bedeutet, wobei der Alkylrest partiell oder vollst. halogeniert sein kann und/oder 1 - 3 der folgenden Reste tragen kann: C₁-C₃-Alkoxy und Cyano.

Des weiteren werden Verbindungen I bevorzugt, in denen R⁴ C₂-C₆-Alkenyl bedeutet.

Des weiteren werden Verbindungen I bevorzugt, in denen R⁴ C₂-C₆-Alkinyl, insbesondere Propargyl, bedeutet.

Des weiteren werden Verbindungen I bevorzugt, in denen R⁴ für Propargyl steht.

Außerdem werden Verbindungen I bevorzugt, in denen R⁴ für trans-3-Chlor-prop-2-enyl steht.

Außerdem werden Verbindungen I bevorzugt, in denen R⁴ für 2-Methoxyethyl steht.

Außerdem werden Verbindungen I bevorzugt, in denen R⁴ für Allyl steht.

Daneben werden Verbindungen der Formel I bevorzugt, in denen die Substituenten aus einer Kombination der oben aufgeführten bevorzugten Substituenten ausgewählt sind.

Insbesondere sind im Hinblick auf ihre Verwendung die in den folgenden Tabellen zusammengestellten Verbindungen der Formeln I.1 bis I.4 bevorzugt.

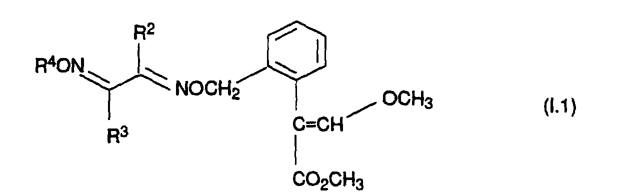

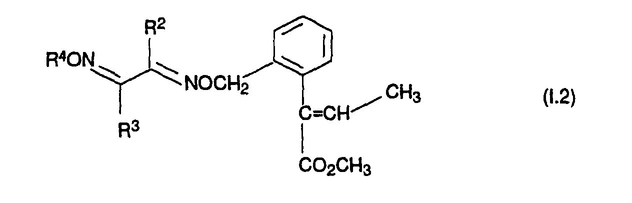

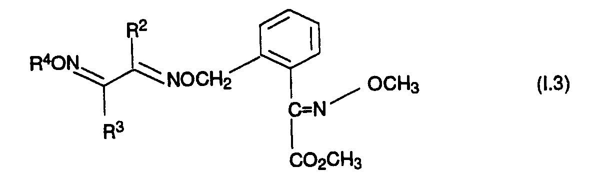

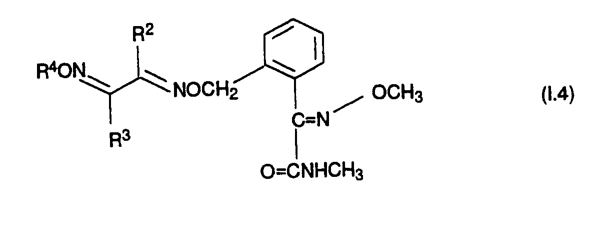

### Tabelle 1

Verbindungen der allgemeinen Formel I.1, in denen R³ Ethenyl bedeutet und die Kombination der Substituenten R² und R⁴ für eine Verbindung jeweils einer Zeile der Tabelle A entspricht

### Tabelle 2

Verbindungen der allgemeinen Formel I.2, in denen R³ Ethenyl bedeutet und die Kombination der Substituenten R² und R⁴ für eine Verbindung jeweils einer Zeile der Tabelle A entspricht

### Tabelle 3

Verbindungen der allgemeinen Formel I.3, in denen R³ Ethenyl bedeutet und die Kombination der Substituenten R² und R⁴ für eine Verbindung jeweils einer Zeile der Tabelle A entspricht

### Tabelle 4

Verbindungen der allgemeinen Formel I.4, in denen R³ Ethenyl bedeutet und die Kombination der Substituenten R² und R⁴ für eine Verbindung jeweils einer Zeile der Tabelle A entspricht

### Tabelle 5

Verbindungen der allgemeinen Formel I.1, in denen R³ Propen-2-yl bedeutet und die Kombination der Substituenten R² und R⁴ für eine Verbindung jeweils einer Zeile der Tabelle A entspricht

### Tabelle 6

Verbindungen der allgemeinen Formel I.2, in denen R³ Propen-2-yl bedeutet und die Kombination der Substituenten R² und R⁴ für eine Verbindung jeweils einer Zeile der Tabelle A entspricht

### Tabelle 7

Verbindungen der allgemeinen Formel I.3, in denen R³ Propen-2-yl bedeutet und die Kombination der Substituenten R² und R⁴ für eine Verbindung jeweils einer Zeile der Tabelle A entspricht

### Tabelle 8

Verbindungen der allgemeinen Formel I.4, in denen R³ Propen-2-yl bedeutet und die Kombination der Substituenten R² und R⁴ für eine Verbindung jeweils einer Zeile der Tabelle A entspricht

### Tabelle 9

Verbindungen der allgemeinen Formel I.1, in denen R³ Propen-1-yl bedeutet und die Kombination der Substituenten R² und R⁴ für eine Verbindung jeweils einer Zeile der Tabelle A entspricht

### Tabelle 10

Verbindungen der allgemeinen Formel I.2, in denen R³ Propen-1-yl bedeutet und die Kombination der Substituenten R² und R⁴ für eine Verbindung jeweils einer Zeile der Tabelle A entspricht

### Tabelle 11

Verbindungen der allgemeinen Formel 1.3, in denen R³ Propen-1-yl bedeutet und die Kombination der Substituenten R² und R⁴ für eine Verbindung jeweils einer Zeile der Tabelle A entspricht

### Tabelle 12

Verbindungen der allgemeinen Formel I.4, in denen R³ Propen-1-yl bedeutet und die Kombination der Substituenten R² und R⁴ für eine Verbindung jeweils einer Zeile der Tabelle A entspricht

### Tabelle 13

Verbindungen der allgemeinen Formel I.1, in denen R³ Isobuten-1-yl bedeutet und die Kombination der Substituenten R² und R⁴ für eine Verbindung jeweils einer Zeile der Tabelle A entspricht

### Tabelle 14

Verbindungen der allgemeinen Formel I.2, in denen R³ Isobuten-1-yl bedeutet und die Kombination der Substituenten R² und R⁴ für eine Verbindung jeweils einer Zeile der Tabelle A entspricht

### Tabelle 15

Verbindungen der allgemeinen Formel I.3, in denen R³ Isobuten-1-yl bedeutet und die Kombination der Substituenten R² und R⁴ für eine Verbindung jeweils einer Zeile der Tabelle A entspricht

### Tabelle 16

Verbindungen der allgemeinen Formel I.4, in denen R³ Isobuten-1-yl bedeutet und die Kombination der Substituenten R² und R⁴ für eine Verbindung jeweils einer Zeile der Tabelle A entspricht

### Tabelle 17

Verbindungen der allgemeinen Formel I.1, in denen R³ Propen-3-yl bedeutet und die Kombination der Substituenten R² und R⁴ für eine Verbindung jeweils einer Zeile der Tabelle A entspricht

### Tabelle 18

Verbindungen der allgemeinen Formel I.2, in denen R³ Propen-3-yl bedeutet und die Kombination der Substituenten R² und R⁴ für eine Verbindung jeweils einer Zeile der Tabelle A entspricht

### Tabelle 19

Verbindungen der allgemeinen Formel I.3, in denen R³ Propen-3-yl bedeutet und die Kombination der Substituenten R² und R⁴ für eine Verbindung jeweils einer Zeile der Tabelle A entspricht

### Tabelle 20

Verbindungen der allgemeinen Formel 1.4, in denen R³ Propen-3-yl bedeutet und die Kombination der Substituenten R² und R⁴ für eine Verbindung jeweils einer Zeile der Tabelle A entspricht

### Tabelle 21

Verbindungen der allgemeinen Formel I.1, in denen R³ Isobuten-3-yl bedeutet und die Kombination der Substituenten R² und R⁴ für eine Verbindung jeweils einer Zeile der Tabelle A entspricht

### Tabelle 22

Verbindungen der allgemeinen Formel I.2, in denen R³ Isobuten-3-yl bedeutet und die Kombination der Substituenten R² und R⁴ für eine Verbindung jeweils einer Zeile der Tabelle A entspricht

### Tabelle 23

Verbindungen der allgemeinen Formel I.3, in denen R³ Isobuten-3-yl bedeutet und die Kombination der Substituenten R² und R⁴ für eine Verbindung jeweils einer Zeile der Tabelle A entspricht

### Tabelle 24

Verbindungen der allgemeinen Formel 1.4, in denen R³ Isobuten-3-yl bedeutet und die Kombination der Substituenten R² und R⁴ für eine Verbindung jeweils einer Zeile der Tabelle A entspricht

### Tabelle 25

Verbindungen der allgemeinen Formel I.1, in denen R³ But-2-en-1-yl bedeutet und die Kombination der Substituenten R² und R⁴ für eine Verbindung jeweils einer Zeile der Tabelle A entspricht

### Tabelle 26

Verbindungen der allgemeinen Formel I.2, in denen R³ But-2-en-1-yl bedeutet und die Kombination der Substituenten R² und R⁴ für eine Verbindung jeweils einer Zeile der Tabelle A entspricht

### Tabelle 27

Verbindungen der allgemeinen Formel I.3, in denen R³ But-2-en-1-yl bedeutet und die Kombination der Substituenten R² und R⁴ für eine Verbindung jeweils einer Zeile der Tabelle A entspricht

### Tabelle 28

Verbindungen der allgemeinen Formel I.4, in denen R³ But-2-en-1-yl bedeutet und die Kombination der Substituenten R² und R⁴ für eine Verbindung jeweils einer Zeile der Tabelle A entspricht

### Tabelle 29

Verbindungen der allgemeinen Formel I.1, in denen R³ But-3-en-1-yl bedeutet und die Kombination der Substituenten R² und R⁴ für eine Verbindung jeweils einer Zeile der Tabelle A entspricht

### Tabelle 30

Verbindungen der allgemeinen Formel I.2, in denen R³ But-3-en-1-yl bedeutet und die Kombination der Substituenten R² und R⁴ für eine Verbindung jeweils einer Zeile der Tabelle A entspricht

### Tabelle 31

Verbindungen der allgemeinen Formel I.3, in denen R³ But-3-en-1-yl bedeutet und die Kombination der Substituenten R² und R⁴ für eine Verbindung jeweils einer Zeile der Tabelle A entspricht

### Tabelle 32

Verbindungen der allgemeinen Formel I.4, in denen R³ But-3-en-1-yl bedeutet und die Kombination der Substituenten R² und R⁴ für eine Verbindung jeweils einer Zeile der Tabelle A entspricht

**Tabelle A**

| Nr. | R² | R⁴ |
|---|---|---|
| A.1 | CH₃ | H |
| A.2 | CH₃ | CH₃ |
| A.3 | CH₃ | C₂H₅ |
| A.4 | CH₃ | n-C₃H₇ |
| A.5 | CH₃ | 1-C₃H₇ |
| A.6 | CH₃ | n-C₄H₉ |
| A.7 | CH₃ | s-C₄H₉ |
| A.8 | CH₃ | i-C₄H₉ |
| A.9 | CH₃ | t-C₄H₉ |
| A.10 | CH₃ | n-C₅H₁₁ |
| A.11 | CH₃ | i-C₅H₁₁ |
| A.12 | CH₃ | neo-C₅H₁₁ |
| A.13 | CH₃ | n-C₆H₁₃ |
| A.14 | CH₃ | n-C₈H₁₇ |
| A.15 | CH₃ | CH₂CH₂Cl |
| A.16 | CH₃ | (CH₂)₄Cl |
| A.17 | CH₃ | CH₂CN |
| A.18 | CH₃ | CH₂CH₂CN |
| A.19 | CH₃ | (CH₂)₃CN |
| A.20 | CH₃ | (CH₂)₄CN |
| A.21 | CH₃ | (CH₂)₆CN |
| A.22 | CH₃ | Cyclohexylmethyl |
| A.23 | CH₃ | 2-Cyclohexyleth-1-yl |
| A.24 | CH₃ | Cyclopropylmethyl |
| A.25 | CH₃ | 2-Cyclopropyleth-1-yl |
| A.26 | CH₃ | 2-Methoxyeth-1-yl |
| A.27 | CH₃ | 2-Ethoxyeth-1-yl |
| A.28 | CH₃ | 2-Isopropoxyeth-1-yl |
| A.29 | CH₃ | 3-Methoxyprop-1-yl |
| A.30 | CH₃ | 3-Ethoxyprop-1-yl |
| A.31 | CH₃ | 3-Isopropoxyprop-1-yl |
| A.32 | CH₃ | 4-Methoxybut-1-yl |
| A.33 | CH₃ | 4-Isopropoxybut-1-yl |
| A.34 | CH₃ | Propen-3-yl |
| A.35 | CH₃ | But-2-en-1-yl |
| A.36 | CH₃ | 3-Methylbut-2-en-1-yl |
| A.37 | CH₃ | 2-Vinyloxyeth-1-yl |
| A.38 | CH₃ | Allyloxyeth-1-yl |
| A.39 | CH₃ | 2-Trifluormethoxyeth-1-yl |
| A.40 | CH₃ | 3-Trifluormethoxyprop-1-yl |
| A.41 | CH₃ | 4-Difluormethoxybut-1-yl |
| A.42 | CH₃ | Methoxycarbonylmethyl |
| A.43 | CH₃ | Aminocarbonylmethyl |
| A.44 | CH₃ | N-Methylaminocarbonylmethyl |
| A.45 | CH₃ | N,N-Dimethylaminocarbonyl-methyl |
| A.46 | CH₃ | 2-Methoxycarbonyleth-1-yl |
| A.47 | CH₃ | 2-Aminocarbonyleth-1-yl |
| A.48 | CH₃ | 2-N-Methylaminocarbonyleth-1-yl |
| A.49 | CH₃ | 2-Dimethylaminocarbonyleth-1-yl |
| A.50 | CH₃ | 2-Aminoeth-1-yl |
| A.51 | CH₃ | 2-Aminoprop-1-yl |
| A.52 | CH₃ | 4-Aminobut-1-yl |
| A.53 | CH₃ | 3-Dimethylaminoprop-1-yl |
| A.54 | CH₃ | 4-Aminothiocarbonylbut-1-yl |
| A.55 | CH₃ | 2-Methoxyiminoprop-1-yl |
| A.56 | CH₃ | 2-Methoxyiminoeth-1-yl |
| A.57 | CH₃ | 6-Aminocarbonylhex-1-yl |
| A.58 | CH₃ | 3-Aminothiocarbonylprop-1-yl |
| A.59 | CH₃ | 2-Aminothiocarbonyleth-1-yl |
| A.60 | CH₃ | Aminothiocarbonylmethyl |
| A.61 | CH₃ | 4-(N,N-Dimethylamino)but-1-yl |
| A.62 | CH₃ | 2-(Methylthio)eth-1-yl |
| A.63 | CH₃ | 2-(Methylsulfonyl)eth-1-yl |
| A.64 | CH₃ | 4-(Methylthio)prop-1-yl |
| A.65 | CH₃ | 4-(Methylsulfonyl)prop-1-yl |
| A.66 | CH₃ | (Z)-3-Chlorbut-2-en-1-yl |
| A.67 | CH₃ | (E)-2-Brombut-2-en-1-yl |
| A.68 | CH₃ | (Z)-2-Brombut-2-en-1-yl |
| A.69 | CH₃ | (E)-1-Chlorprop-1-en-3-yl |
| A.70 | CH₃ | (Z)-1-Chlorprop-1-en-3-yl |
| A.71 | CH₃ | But-1-en-3-yl |
| A.72 | CH₃ | CH₂CH₂Br |
| A.73 | CH₃ | CH₂CH₂CH₂Br |
| A.74 | CH₃ | CH₂CH₂CH₂CH₂Br |
| A.75 | CH₃ | 2-Methoxyprop-1-yl |
| A.76 | CH₃ | 2-Ethoxyprop-1-yl |
| A.77 | CH₃ | 2-Isopropoxyprop-1-yl |
| A.78 | CH₃ | But-1-in-3-yl |
| A.79 | CH₃ | But-1-in-4-yl |
| A.80 | CH₃ | But-2-in-4-yl |
| A.81 | CH₃ | Pent-1-in-4-yl |
| A.82 | CH₃ | Pent-2-in-4-yl |
| A.83 | CH₃ | Pent-1-in-5-yl |
| A.84 | CH₃ | Pent-2-in-5-yl |
| A.85 | CH₃ | E-2-Chlorbut-2-en-1-yl |
| A.86 | CH₃ | Z-2-Chlorbut-2-en-1-yl |
| A.87 | CH₃ | Z-1-Chlorprop-1-en-3-yl |
| A.88 | CH₃ | 2-Methylprop-1-en-3-yl |
| A.89 | CH₃ | 2-Chlorprop-1-en-3-yl |
| A.90 | CH₃ | 2-Bromprop-1-en-3-yl |
| A.91 | CH₃ | But-2-en-1-yl |
| A.92 | CH₃ | But-3-en-1-yl |
| A.93 | CH₃ | 1-Iodprop-1-in-3-yl |
| A.94 | CH₃ | E-3-Brombut-2-en-1-yl |
| A.95 | CH₃ | Z-3-Brombut-2-en-1-yl |

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten, Phycomyceten und Basidiomyceten, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Gemüse und Zierpflanzen, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I eignen sich außerdem zur Bekämpfung von Schadpilzen im Materialschutz (z.B. Holz, Papier, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindungen gewährleisten. Die Formulierungen werden in bekannter Weise hergestellt, z.B. durch Verstrecken des Wirkstoffs mit Lösungsmitteln und/ oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-diisopropylester;
heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecylmorpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidinmethanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
   sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Strobilurine wie Methyl-E-methoxyimino-[α-(o-tolyloxy)-o-tolyl]-acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid.

Anilinopyrimidine wie N-(4,6-Dimethylpyrimidin-2-yl)-anilin, N-[4-Methyl-6-(1-propinyl)-pyrimidin-2-yl]-anilin, N-(4-Methyl-6-cyclopropyl-pyrimidin-2-yl)-anilin.

Phenylpyrrole wie 4-(2,2-Difluor-1,3-benzodioxol-4-yl)-pyrrol-3-carbonitril.

Zimtsäureamide wie 3-(4-Chlorphenyl)-3-(3,4-dimethoxyphenyl)acrylsäuremorpholid.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, A1kylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält maneine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1: 2-[[[[2-(Isopropyl)-1-(methyl)-2-(oxo)ethyliden]amino]oxy] methyl]-α-(methoxymethylen)-phenylessigsäuremethylester (nicht erfindungsgemäß)

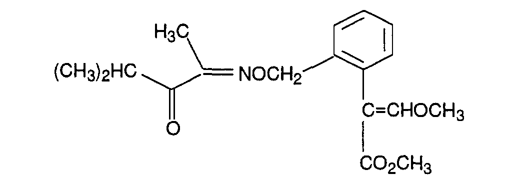

Zu einer Lösung aus 8 g (62 mmol) 1-Isopropyl-1,2-propandion-2-oxim in 50 ml N,N-Dimethylformamid gibt man 11 g einer 30 %igen methanol. Natriummethylatlösung. Man läßt 30 Minuten bei Raumtemperatur rühren, tropft dann eine Lösung aus 18,6 g (68 mmol) 2-(Brommethyl)-α-(methoxymethylen)phenylessigsäuremethylester in 50 ml N,N-Dimethylformamid zu und rührt ca. 1 Stunde bei 40°C. Anschließend wird die Reaktionslösung mit Wasser versetzt und mit Methyl-tert.butylether extrahiert. Trocknung der organischen Extrakte über Natriumsulfat und Einengen am Rotationsverdampfer liefert 18 g eines gelben Feststoffs. Nach Umkristallisation aus Hexan, Absaugen und Trocknung im Vakuumtrockenschrank erhält man 15 g (73 %) der Titelverbindung in Form eines leicht gelblich gefärbten Feststoffs (Smp. 67-69°C)
IR [cm⁻¹] (KBr):
768, 997, 1009, 1109, 1127, 1210, 1254, 1630, 1683, 1699, 2950.

### Beispiel 2 2-[[[[2-(Allyloxy)imino-2-(isopropyl)-1-(methyl)ethyliden] amino]oxy]methyl]-α-(methoxymethylen)-phenylessigsäuremethylester (nicht erfindungsgemäß)

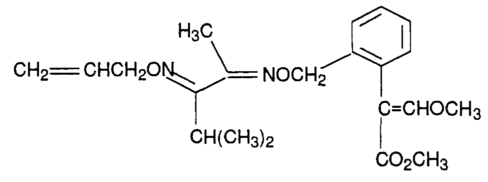

Zu einer Lösung aus 1,5 g (4,5 mmol) 2-[[[[2-(Isopropyl)-1-(methyl)-2-(oxo)ethyliden]amino]oxy]methyl]-α-(methoxymethylen)phenylessigsäuremethylester in 60 ml Methanol gibt man 0,7 g (9mmol) o-Allyl-hydroxylamin und 0,9 g (4,5mmol) p-Toluol-Sulfonsäure. Man läßt die Reaktionslösung 4 Tage bei Raumtemperatur stehen, versetzt anschließend mit Wasser und extrahiert mit Methyl-tert.butylether. Die vereinigten organischen Extrakte werden über Natriumsulfat getrocknet und eingeengt. Nach Säulenchromatographie an Kieselgel (Methyl-tert.-butylether/Hexan) erhält man die Titelverbindung in Form zweier Isomere [Isomerie in der Seitenkette, (Z, E) bzw. (E, E)].
1. Isomer (0,3 g; leicht gelbliches Öl)
   ¹H-NMR (CDCl₃):
   - δ =: 1,16 (d, 6H); 1,99 (s, 3H); 3,50 (m, 1H); 3,67 (s, 3H); 3,81 (s, 3H); 4,60 (d, 2H); 5,08 (s, 2H); 5,25 (m, 2H); 5,97 (m, 1H); 7,12-7,46 (m, 4H); 7,56 (s, 1H) ppm.
2. Isomer (0,6 g; weißer Feststoff; Smp 58-60°C)
   ¹H-NMR (CDCl₃):
   - δ =: 1,08 (d, 6H); 2,00 (s, 3H); 2,71 (m, 1H); 3,67 (s, 3H); 3,81 (s, 3H); 4,53 (d, 2H); 5,06 (s, 2H); 5,21 (m, 2H); 5,96 (m, 1H); 7,12-7,46 (m, 4H); 7,56 (s, 1H) ppm.

### Beispiel 3 2-[[[[2-(Isopropyl)-1-(methyl)-2-(oxo)ethyliden]amino]oxy]-methyl]-α-(ethyliden)-phenylessigsäuremethylester (nicht erfindungsgemäß)

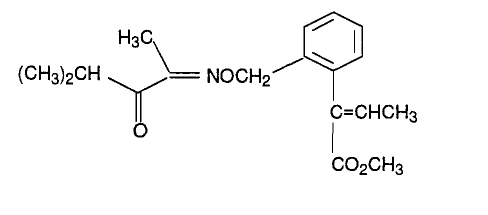

Eine Lösung von 9 g (33 mmol) 2-(Brommethyl)-α-ethylidenephenylessigsäuremethylester in 50 ml N,N-Dimethylformamid wird mit 9,2 g (67 mmol) Kaliumcarbonat versetzt und nach Zugabe von 4,3 g 1-Isopropyl-1,2-propandion-2-oxim 2 Stunden bei 50°C gerührt.Anschließend gibt man das Reaktionsgemisch auf Wasser und extrahiert mit Methyl-tert.butylether. Die vereinigten organischen Extrakte werden mit Wasser gewaschen, über Natriumsulfat getrocknet und eingeengt. Nach Säulenchromatographie an Kieselgel (Cyclohexan/Essigsäureethylester) erhält man 7,8 g der Titelverbindung als gelbliches Öl.
¹H-NMR (CDCl₃):
- δ =: 1,06 (d, 6H); 1,62 (d, 3H); 1,90 (s, 3H); 3,86 (m, 1H); 3,70 (s, 3H); 5,13 (s, 2H); 7,08-7,50 (m, 4H) ppm.

### Beispiel 4 2-[[[[2-(Isopropyl)-2-(methoxyimino)-1-(methyl) ethyliden] amino] oxy]methyl]-α-(ethyliden)-phenylessigsäuremethylester (nicht erfindungsgemäß)

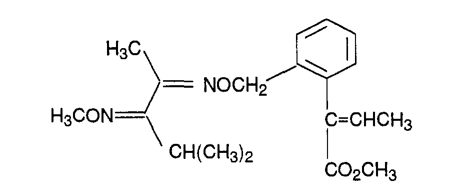

Zu einer Lösung von 2 g (6,3 mmol) 2-[[[[2-(Isopropyl)-1-(methyl)-2-(oxo)ethyliden]amino]oxy]methyl]-α-(ethyliden)-phenylessigsäuremethylester in 50 ml Methanol gibt man 7,9 g einer 20 %igen methanol. O-Methylhydroxylamin-Hydrochlorid-Lösung sowie 2-3 g Molsieb (3Å). Man läßt die Reaktionslösung 3 Tage bei Raumtemperatur stehen und rührt dann 4 Stunden bei 40°C. Nach Abfiltrieren des Molekularsiebes gibt man die Lösung auf Eiswasser und extrahiert mit Methyl-tert.butylether. Die vereinigten organischen Extrakte werden mit Wasser gewaschen, über Natriumsulfat getrocknet und eingeengt. Nach Säulenchromatographie an Kieselgel (Cyclohexan/Essigsäureethylester) erhält man 1,7 g (78 %) der Titelverbindung in Form eines Isomerengemisches (Isomerie in der Seitenkette, Isomere 1:2) als gelbliches Öl.
IR [cm⁻¹] (Film) :
761, 907, 1013, 1038, 1208, 1252, 1435, 1719, 2937, 2963.

### Beispiel 5 (E,E,E)-2-[[[[2-(Isopropyl)-2-(methoxyimino)-1-(methyl)-ethyliden]amino]oxy]methyl]-α-(methoxyimino)phenylessigsäuremethylester (nicht erfindungsgemäß)

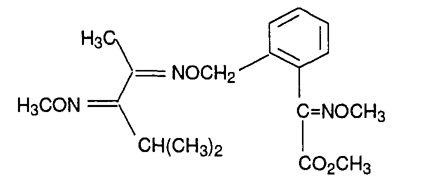

Zu einer Lösung aus 49,7 g (315 mmol) (E,E)-1-(Isopropyl)-1-(methoxyimino-propan-2-on-2-oxim in 400 ml N,N-Dimethylformamid gibt man 1 eq Natriummethylat (30%-ige methanol. Lösung) und läßt 30 min. bei Raumtemperatur rühren. Anschließend tropft man eine Lösung aus 82,8 g (290 mmol) 2-(Brommethyl)-α-methoxyiminophenylessigsäuremethylester in 200 ml N,N-Dimethylformamid zu. Man läßt 1 Stunde bei Raumtemperatur rühren und gießt die Reaktionslösung anschließend auf Eiswasser. Der ausgefallene Feststoff wird abgesaugt. Nach mehrmaligem Waschen mit Wasser und Pentan erhält man 94,5 g eines kristallinen Feststoffs vom Schmelzpunkt 92-94°C.
¹H-NMR (CDCl₃):
- δ =: 1,12 (d, 6H); 1,96 (s, 3H); 3,45 (m, 1H); 3,84 (s, 3H); 3,89 (s, 3H); 4,04 (s, 3H); 5,05 (s, 2H); 7,15-7,48 (m, 4H) ppm.

### Beispiel 6 (E,E,E)-2-[[[[2-(Isopropyl)-2-(methoxyimino)-1-(methyl)-ethyliden]amino]oxy]methyl]-α-(methoxyimino)phenylessigsäuremonomethylamid (nicht erfindungsgemäß)

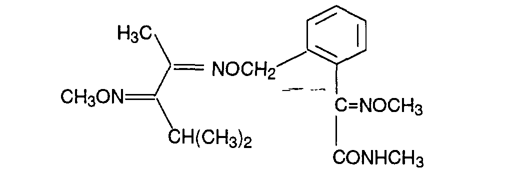

50 g (138 mmol) (E,E,E)-2-[[[[2-(Isopropyl)-2-(methoxyimino) -1-(methyl)ethyliden]amino]oxy]methyl]-α-(methoxyimino)-phenylessigsäuremethylester werden in 500 ml Tetrahydrofuran gelöst, mit 107 g 40 %iger wäßriger Monomethylaminlösung versetzt und 30-60 min. bei 50°C gerührt. Anschließend wird mit Wasser versetzt und mit Methyl-tert.-butylether extrahiert. Die organ. Phase wird mit Wasser gewaschen, über Na₂SO₄ getrocknet und eingeengt. Man erhält 41 g eines braunen Feststoffs, der zur Reinigung in Pentan suspendiert wird. Nach Absaugen, Waschen des Rückstandes und Trocknung isoliert man 37 g der Titelverbindung in Form farbloser Kristalle. Smp: 78-79°C.
¹H-NMR (CDCl₃):
- δ =: 1,13 (d, 6H); 1,95 (s, 3H); 2,88 (d, 3H); 3,46 (m, 1H) ; 3,89 (s, 3H); 3,94 (s, 3H); 5,06 (s, 2H); 6,72 (s breit, 1H); 7,15-7,46 (m, 4H) ppm.

### Beispiel 7 2-[[[[2-(Methoxyimino)-1-(methyl)-2-(phenoxymethyl) -ethyliden] amino]oxy]methyl]-α-(methoxyimino)-phenylessigsäuremethylester (nicht erfindungsgemäß)

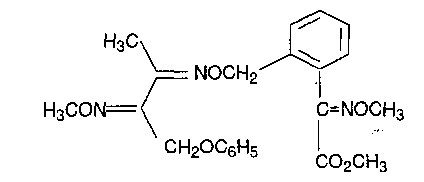

Zu einer Lösung aus 2 g 2-[[[[1-(Methyl)-2-(oxo)-2-(phenoxymethyl)-ethyliden]amino]oxy]methyl]-α-(methoxyimino)-phenylessigsäuremethylester in 5 ml Methanol gibt man 0,4 g O-Methyl-hydroxylaminhydrochlorid und 1,2 g Pyridin. Man läßt die Reaktionslösung 60 Minuten bei Raumtemperatur rühren, nimmt den Rückstand in Dichlormethan auf und wäscht die organ. Phase mit verdünnter Salzsäure und mit Wasser. Die organische Phase wird über Natriumsulfat getrocknet und eingeengt. Kritallisation des Rückstands aus Pentan liefert 0,7 g der Titelverbindung in Form leicht rötlicher Kristalle (Fp 81-83°C).

### Beispiel 8 2-[[[[2-(Methoxyimino)-1-(methyl)-2-(phenoxymethyl)-ethyliden]-amino]oxy]methyl]-α-(methoxyimino)-phenylessigsäuremonomethylamid (nicht erfindungsgemäß)

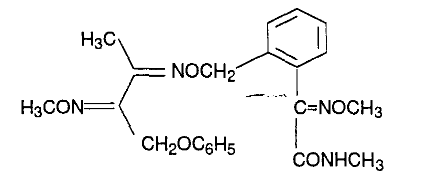

0,5 g 2-[[[[2-(Methoxyimino)-1-(methyl)-2-(phenoxymethyl)ethyliden]amino]oxy]methyl]-α-(methoxyimino)-phenylessig-säuremethylester werden in 5 ml Tetrahydrofuran gelöst, mit 0,5 g 40 %iger wäßriger Monomethylaminlösung versetzt und bei Raumtemperatur gerührt. Das Reaktionsgemisch wird eingeengt und der Rückstand mit Pentan verrieben. Nach Abfiltrieren des Niederschlags und Waschen mit Pentan erhält man 0,5 g der Titelverbindung in Form leicht rosafarbene Kristalle (Fp 136-138°C).

### Beispiel 9 2-[[[[2-(Brommethyl)-1-(methyl)-2-(oxo)ethyliden]amino]-oxy]methyl]-α-(methoxyimino)-phenylessigsäuremethylester (nicht erfindungsgemäß)

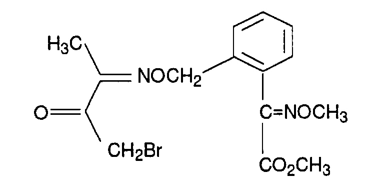

Zu einer Lösung aus 39,8 g 2-[[[[1,2-(Dimethyl)-2-(oxo)ethyliden]amino]oxy]-methyl]-α-(methoxyimino)-phenylessigsäuremethylester in 200 ml Chloroform werden bei 40°C zunächst 5 ml einer Lösung von 20,8 g Brom in 20 ml Chloroform getropft. Nach Anspringen der Reaktion wird die restliche Bromlösung derart zugetropft, daß die Temperatur bei 40-50°C gehalten werden kann. Nach Abreagieren des Broms (Entfärbung, Temperaturabfall) wird der Reaktionsverlauf dünnschichtchromatographisch verfolgt. Liegt noch Ausgangsverbindung vor, so wird bis zur vollständigen Umsetzung weitere Bromlösung zugetropft (40°C, DC-Kontrolle). Man läßt das Reaktionsgemisch auf Raumtemperatur abkühlen, versetzt mit Wasser und neutralisiert mit Natriumhydrogencarbonatlösung. Die organische Phase wird abgetrennt, mit Natriumhydrogencarbonatlösung und mit Wasser gewaschen, über Natriumsulfat getrocknet und eingeengt. Man erhält die Titelverbindung in Form eines dunklen Öls (50 g). Das Rohprodukt kann ohne vorherige Reinigung weiter umgesetzt werden.
¹H-NMR (CDCl₃):
- δ [ppm] =: 1,94 (s,3H); 3,86 (s,3H); 4,04 (s,3H); 4,35 (s,2H); 5,18 (s,2H); 7,15-7,0 (m,4H)

### Beispiel 10 2-[[[[2-(4-Chlorphenoxymethyl)-1-methyl-2-oxo-ethyliden]-amino]oxy]methyl]-α-(methoxyimino-phenylessigsäuremethylester (nicht erfindungsgemäß)

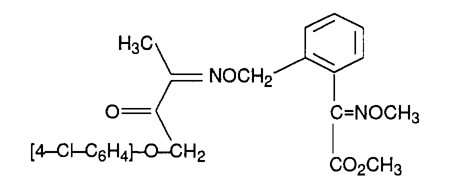

Eine Lösung aus 21,9 g 2-[[[[2-(Brommethyl)-1-(methyl)-2-(oxo)ethyliden]amino]oxy]methyl]-α-(methoxyimino)-phenylessigsäuremethylester in 150 ml Aceton wird mit 15,7 g Kaliumcarbonat und 11 g 4-Chlorphenol versetzt und 24 h bei Raumtemperatur gerührt. Man engt das Reaktionsgemisch am Rotationsverdampfer ein und nimmt den verbleibenden Rückstand in Essigester auf. Die organische Phase wird mit Wasser gewaschen, über Na₂SO₄ getrocknet und eingeengt. Nach säulenchromatographischer Reinigung an Kieselgel erhält man die Titelverbindung in Form eines kristallinen Feststoffs (10,8 g). Fp: 110-114°C.

### Beispiel 11 2-[[[[2-(4-Chlorphenoxymethyl)-2-(ethoxyimino)-1-(methyl)-ethyliden]amino]oxymethyl]-α-(methoxyimino)-phenylessigsäuremethylester (nicht erfindungsgemäß)

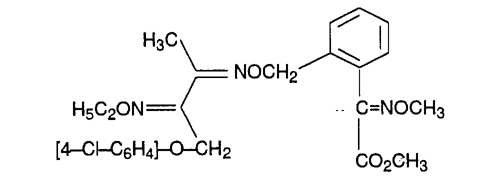

Zu einer Lösung aus 2,5 g 2-[[[[2-(4-Chlorphenoxymethyl)-1-(methyl)-2-(oxo)ethyliden]amino]oxy]methyl]-α-(methoxyimino)-phenylessigsäuremethylester in 5 ml Methanol gibt man 0,6 g O-Ethyl-hydroxylaminhydrochlorid und 1,4 g Pyridin. Man läßt die Reaktionslösung 18 Stunden bei Raumtemperatur rühren, nimmt das Gemisch in Dichlormethan auf, wäscht mit verdünnter Salzsäure und mit Wasser. Nach Einengen am Rotationsverdampfer erhält man 2,7 g der Titelverbindung in Form eines kristallinen Feststoff. Fp: 84-87°C.

### Beispiel 12 3-(Methoxyimino)-1-(phenyl)-pent-1-in-4-on-4-oxim (nicht erfindungsgemäß)

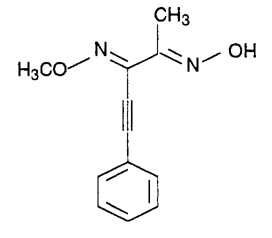

Eine Lösung aus 1,6 g 1-(Phenyl)-pent-1-in-3,4-dion-4-oxim in 20 ml Methanol wird mit 2 g Pyridin und 4,9 g einer 18%-igen methanolischen O-Methylhydroxylamin-Hydrochlorid-Lösung versetzt und 24h bei Raumtemperatur (ca. 25°C) gerührt. Man nimmt die Reaktionslösung in tert.-Butyl-methylether auf und versetzt mit 10%-iger Salzsäure. Die wäßrige Phase wird mit tert.-Butyl-methylether extrahiert. Anschließend werden die organischen Phasen mit 10%-iger Salzsäure und mit Wasser gewaschen, über Na₂SO₄ getrocknet und das Lösungsmittel bei vermindertem Druck abdestilliert. Man erhält 1,4 g der Titelverbindung als farblosen Feststoff (76% Ausbeute); Smp.: 140-144°C.

### Beispiel 13 2-[[[[2-(Methoxyimino)-1-(methyl)-2-(phenylethin-1-yl)-ethyliden]-amino]oxy]methyl]-α-(methoxyimino)phenylessigsäuremethylester (nicht erfindungsgemäß)

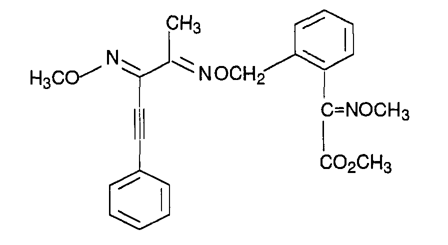

Eine Lösung aus 1,4 g 1-(Methoxyimino)-1-(phenylethin-1-yl)-propan-2-on-2-oxim in 20 ml N,N-Dimethylformamid wird mit 1,4 g einer 30%-igen methanolischen Natrium-methylat-Lösung versetzt und 10 min. bei Raumtemperatur gerührt. Anschließend tropft man eine Lösung aus 2.1 g 2-(Brommethyl)-α-methoxyiminophenylessigsäuremethylester in 20 ml N,N-Dimethylformamid zu. Man läßt 1h bei Raumtemperatur rühren, gießt die Reaktionsmischung auf Wasser und extrahiert mit tert.-Butylmethylether. Die organische Phase wird mit Wasser gewaschen, über Na₂SO₄ getrocknet, abfiltriert und bei vermindertem Druck eingeengt. Kristallisation des öligen Rückstands aus Methanol liefert 1,9 g der Titelverbindung (60% Ausbeute); Smp.: 100-104°C.

### Beispiel 14 2-[[[[2-(Methoxyimino)-1-(methyl)-2-(phenylethin-1-yl)-ethyliden]-amino]oxy]methyl]-α-(methoxyimino)phenylessigsäuremonomethylamid (nicht erfindungsgemäß)

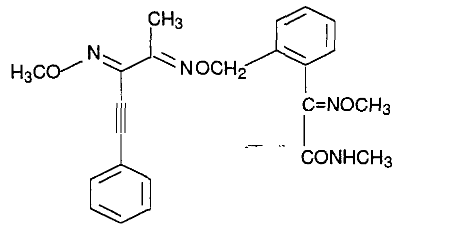

1g des Esters aus Beispiel 13 wird in 50 ml Tetrahydrofuran gelöst, mit 1,4 g 40%-iger wäßriger Monomethylamin-Lösung versetzt und 20h bei Raumtemperatur gerührt. Anschließend gibt man die Reaktionsmischung auf Wasser und extrahiert mit tert.-Butylmethylether. Die organische Phase wird mit Wasser gewaschen, über Na₂SO₄ getrocknet, filtriert und bei vermindertem Druck eingeengt. Kristallisation des öligen Rückstands aus Methanol liefert 0,8 g der Titelverbindung als Feststoff (79% Ausbeute); Smp.: 131-133°C.

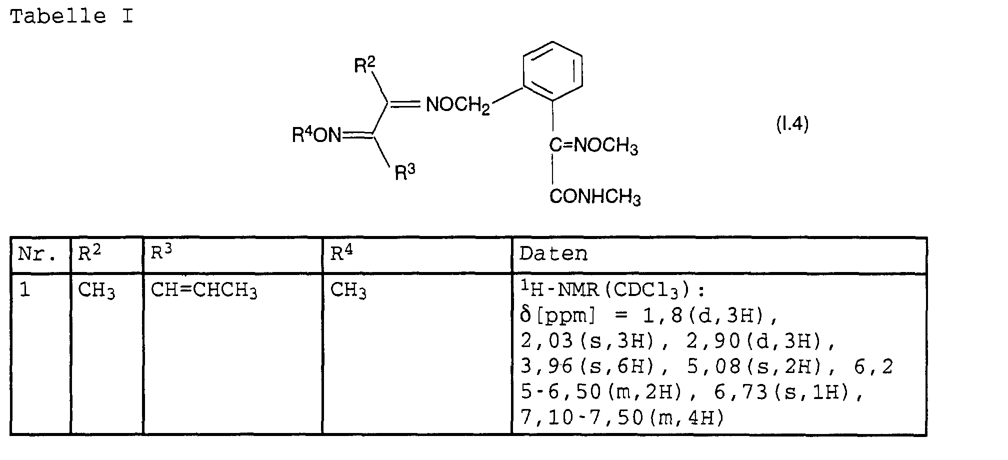

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Botrytis cinerea (Grauschimmel)

Scheiben von grünen Paprikaschoten wurden mit der Wirkstoffaufbereitung tropfnaß gespritzt. Nach dem Abtrocknen wurden die Fruchtscheiben mit einer Sporensuspension des Pilzes *Botrytis cinerea* (1,7·10⁶ Sporen pro ml einer 2 %-igen Biomalzlösung) besprüht und 4 Tage bei 18°C bei hoher Luftfeuchtigkeit bewahrt. Die Auswertung erfolgte visuell.

In diesem Test zeigten die mit 250 ppm der Verbindung der Tabelle I behandelten Pflanzen einen Befall von 40% und weniger, während die unbehandelten (Kontroll-) Pflanzen zu 100% befallen waren.

### Wirkung gegen Erysiphe graminis var. tritici (Weizenmehltau)

Blätter von Weizenkeimlingen (Sorte "Frühgold") wurden zunächst mit der wäßrigen Aufbereitung der Wirkstoffe behandelt. Nach ca. 24 h wurden die Pflanzen mit Sporen des Weizenmehltaus (*Erysiphe graminis var*. *tritici*) bestäubt. Die so behandelten Pflanzen wurden anschließend für 7 Tage bei 20 bis 22°C und einer relativen Luftfeuchtigkeit von 75 bis 80 % inkubiert. Anschließend wurde das Ausmaß der Pilzentwicklung ermittlelt.

In diesem Test zeigten die mit 16 ppm der Verbindung der Tabelle I behandelten Pflanzen einen Befall von 15% und weniger, während die unbehandelten (Kontroll-) Pflanzen zu 80% befallen waren.

### Wirkung gegen Puccinia recondita (Weizenbraunrost)

Blätter von Weizensämlingen (Sorte "Kanzler") wurden mit Sporen des Braunrosts (*Puccinia recondita)* bestäubt. Die so behandelten Pflanzen wurden 24 h bei 20 bis 22°C und einer relativen Luftfeuchtigkeit von 90 bis 95% inkubiert und anschließend mit der wäßrigen Wirkstoffaufbereitung behandelt. Nach weiteren 8 Tagen bei 20 bis 22°C und 65 bis 70% relativer Luftfeuchtigkeit wurde das Ausmaß der Pilzentwicklung ermittlelt. Die Auswertung erfolgte visuell.

In diesem Test zeigten die mit 63 ppm der Verbindung der Tabelle I behandelten Pflanzen einen Befall von 15% und weniger, während die unbehandelten (Kontroll-) Pflanzen zu 70% befallen waren.

### Wirkung gegen Pyricularia oryzae (Reisbrand)

Reiskeimlinge (Sorte: "Tai Nong 67") wurden mit der Wirkstoffaufbereitung tropfnaß gespritzt. Nach 24 Stunden wurden die Pflanzen mit einer wäßrigen Sporensuspension des Pilzes *Pyricularia oryzae* besprüht und 6 Tage bei 22 bis 24°C bei einer relativen Luftfeuchtigkeit von 95 bis 99% bewahrt. Die Beurteilung erfolgte visuell.

In diesem Test zeigten die mit 63 ppm der Verbindung der Tabelle I behandelten Pflanzen einen Befall von 15% und weniger, während die unbehandelten (Kontroll-) Pflanzen zu 80% befallen waren.

### Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 bis 100 %-ige Hemmung bzw. Mortalität hervorriefen (Wirkschwelle bzw. Minimalkonzentration).

## Patentansprüche

1. Phenylessigsäurederivate der Formel I 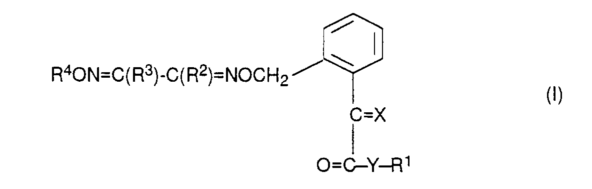 in der die Substituenten und der Index die folgende Bedeutung haben:
X NOCH₃, CHOCH₃ und CHCH₃;
Y NR und O;
R, R¹ Wasserstoff und C₁-C₄-Alkyl;
R² Methyl;
R³ C₂-C₆-Alkenyl;
R⁴ Wasserstoff,
C₁-C₆-Alkyl, C₂-C₆-Alkenyl, C₂-C₆-Alkinyl, wobei die Reste partiell oder vollständig halogeniert sein können und/oder 1 - 3 der folgenden Substituenten tragen können: Cyano, Hydroxy, Amino, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, Aminocarbonyl, C₁-C₆-Alkylaminocarbonyl, Di-C₁-C₆-alkylaminocarbonyl, Alkenyloxy, C₃-C₆-Cycloalkyl, C₃-C₆-Cycloalkyloxy, wobei die cyclischen Gruppen ihrerseits partiell oder vollständig halogeniert sein können oder einen bis drei der Reste C₁-C₃-Alkyl oder C₁-C₃-Alkoxy tragen können;
sowie deren Salze.

2. Phenylessigsäurederivate der Formel I nach Anspruch 1, in der
R³ Vinyl, Allyl, Prop-1-enyl, 2-Methylprop-1-enyl oder iso-Propenyl bedeutet.

3. Verbindungen der Formel I gemäß Anspruch 1, in denen X NOCH₃, Y NH und R¹ Methyl bedeuten.

4. Verbindungen der Formel I gemäß Anspruch 1, in denen R¹ für Methyl steht.

5. Eine Verbindung der Formel

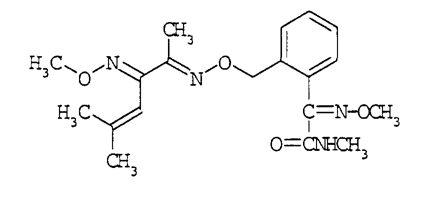

6. Eine Verbindung der Formel

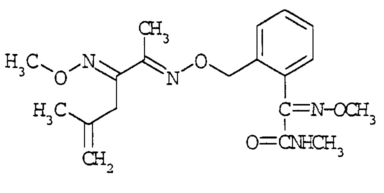

7. Eine Verbindung der Formel

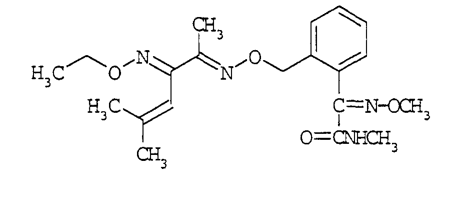

8. Verfahren zur Herstellung der Verbindungen der Formel I gemäß Anspruch 1, **dadurch gekennzeichnet, daß** man ein Benzylderivat der Formel II 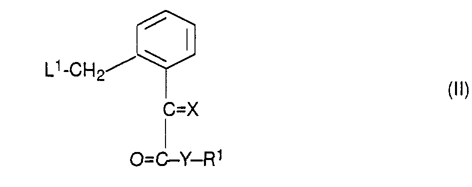 in der L¹ für eine nucleophil austauschbare Abgangsgruppe steht, in an sich bekannter Weise mit einem Hydroxyimin der Formel III
R⁴ON=C(R³)-C(R²)=NOH (III)
umsetzt.

9. Verfahren zur Herstellung der Verbindungen der Formel I gemäß Anspruch 1, **dadurch gekennzeichnet, daß** man ein Benzylderivat der Formel II gemäß Anspruch 8 in an sich bekannter Weise mit einem Dihydroxyimin der Formel IV
HON=C(R³)-C(R²)=NOH (IV)
zu einer Verbindung der Formel V 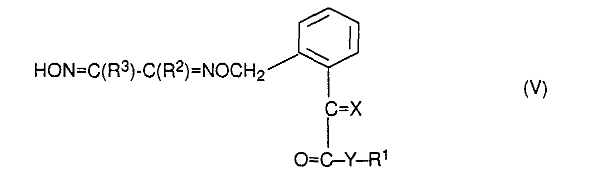 umsetzt und V anschließend mit einer Verbindung der Formel VI
R⁴-L² (VI)
in der L² für eine nucleophil austauschbare Abgangsgruppe steht, zu I umsetzt.

10. Verfahren zur Herstellung der Verbindungen der Formel I gemäß Anspruch 1, **dadurch gekennzeichnet, daß** man ein Benzylderivat der Formel II gemäß Anspruch 8 in an sich bekannter Weise mit einem Carbonylhydroxyimin der Formel VII
O=C(R³)-C(R²)=NOH (VII)
zu einer Verbindung der Formel VIII 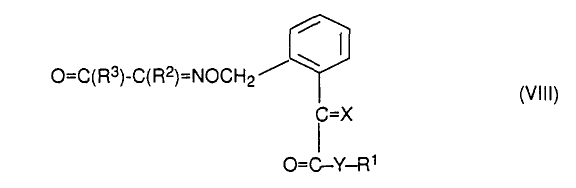 umsetzt, VIII anschließend entweder
a) zunächst mit Hydroxylamin oder dessen Salz und danach mit einer Verbindung der Formel VI (R⁴-L²) gemäß Anspruch 9 oder
b) mit einem Hydroxylamin oder einem Hydroxylammoniumsalz der Formel IXa bzw. IXb 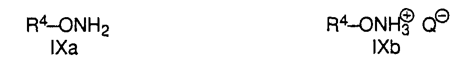 in der Q^{⊖} für das Anion einer Säure steht,
zu I umsetzt.

11. Verbindungen der allgemeinen Formel III gemäß Anspruch 8.

12. Verbindungen der allgemeinen Formel IV gemäß Anspruch 9.

13. Verbindungen der allgemeinen Formel V gemäß Anspruch 9.

14. Verbindungen der allgemeinen Formel VIII gemäß Anspruch 10.

15. Mittel gegen tierische Schädlinge oder Schadpilze, enthaltend übliche Zusatzstoffe und eine wirksame Menge einer Verbindung der Formel I gemäß Anspruch 1.

16. Verfahren zur Bekämpfung von tierischen Schädlingen oder Schadpilzen, **dadurch gekennzeichnet, daß** man die Schädlinge oder Schadpilze, deren Lebensraum oder die von ihnen freizuhaltenden Pflanzen, Flächen, Materialien oder Räume mit einer wirksamen Menge einer Verbindung der Formel I gemäß Anspruch 1 behandelt.

17. Verwendung der Verbindungen I gemäß Anspruch 1 zur Herstellung von Mitteln gegen tierische Schädlinge oder Schadpilze.

18. Verwendung der Verbindungen I gemäß Anspruch 1 zur Bekämpfung von tierischen Schädlingen oder Schadpilzen.

## Claims

1. A phenylacetic acid derivative of the formula I 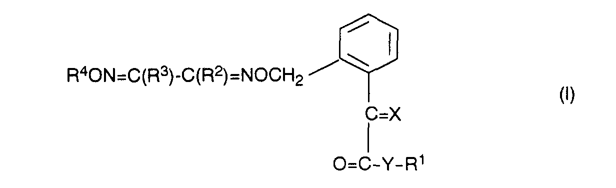 where the substituents and the index have the following meanings:
X is NOCH₃, CHOCH₃ and CHCH₃;
Y is NR and O;
R, R¹ are hydrogen and C₁-C₄-alkyl;
R² is methyl;
R³ is C₂-C₆-alkenyl;
R⁴ is hydrogen,
C₁-C₆-alkyl, C₂-C₆-alkenyl, C₂-C₆-alkynyl, it being possible for these radicals to be partially or fully halogenated and/or to have attached to them one to three of the following substituents: cyano, hydroxyl, amino, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, aminocarbonyl, C₁-C₆-alkylaminocarbonyl, di-C₁-C₆-alkylaminocarbonyl, alkenyloxy, C₃-C₆-cycloalkyl, C₃-C₆-cycloalkyloxy, it being possible for these cyclic groups for their part to be partially or fully halogenated or to have attached to them one to three of the radicals C₁-C₃-alkyl or C₁-C₃-alkoxy;
and a salt thereof.

2. A phenylacetic acid derivative of the formula I as claimed in claim 1, where
R³ is vinyl, allyl, prop-1-enyl, 2-methylprop-1-enyl or iso-propenyl.

3. A compound of the formula I as claimed in claim 1 where X is NOCH₃, Y is NH and R¹ is methyl.

4. A compound of the formula I as claimed in claim 1 where R¹ is methyl.

5. A compound of the formula

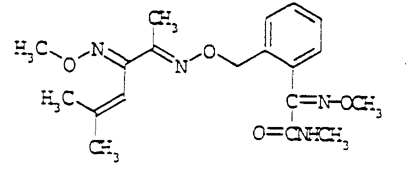

6. A compound of the formula

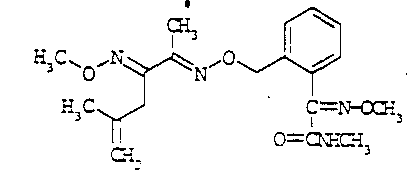

7. A compound of the formula

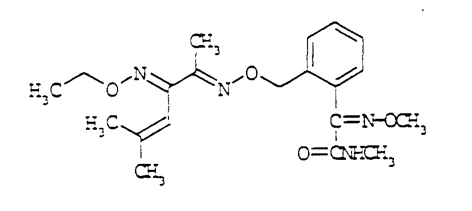

8. A process for the preparation of the compounds of the formula I as claimed in claim 1, which comprises reacting a benzyl derivative of the formula II 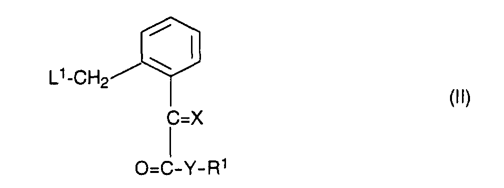 where L¹ is a nucleophilically exchangeable leaving group in a manner known per se with a hydroxyimine of the formula III
R⁴ON=C(R³)-C(R²)=NOH (III).

9. A process for the preparation of the compounds of the formula I as claimed in claim 1, which comprises reacting a benzyl derivative of the formula II as claimed in claim 8 in a manner known per se with a dihydroxyimine of the formula IV
HON=C(R³)-C(R²)=NOH (IV)
to give a compound of the formula V 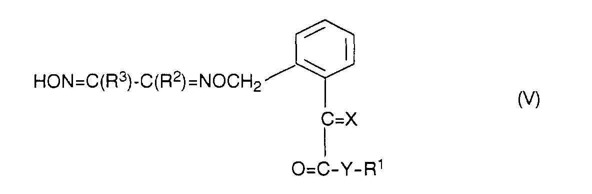 and subsequently reacting V with a compound of the formula VI
R⁴-L² (VI)
where L² is a nucleophilically exchangeable leaving group, to give I.

10. A process for the preparation of the compounds of the formula I as claimed in claim 1, which comprises reacting a benzyl derivative of the formula II as claimed in claim 8 in a manner known per se with a carbonylhydroxyimine of the formula VII
O=C(R³)-C(R²)=NOH (VII)
to give a compound of the formula VIII 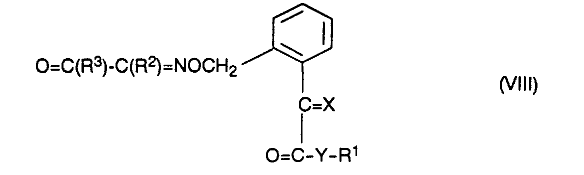 and subsequently reacting VIII either
a) first with hydroxylamine or a salt thereof and then with a compound of the formula VI (R⁴-L²) as claimed in claim 9 or
b) with a hydroxylamine or a hydroxylammonium salt of the formula IXa or IXb 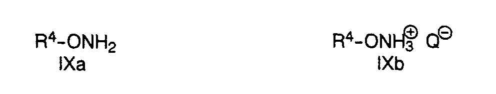 where Q^{⊖} is the anion of an acid,
to give I.

11. A compound of the general formula III as claimed in claim 8.

12. A compound of the general formula IV as claimed in claim 9.

13. A compound of the general formula V as claimed in claim 9.

14. A compound of the general formula VIII as claimed in claim 10.

15. A composition against animal pests or harmful fungi, comprising customary additives and an effective amount of a compound of the formula I as claimed in claim 1.

16. A method of controlling animal pests or harmful fungi, which comprises treating the pests or harmful fungi, their environment, or the plants, areas, materials or spaces to be kept free from them with an effective amount of a compound of the formula I as claimed in claim 1.

17. The use of the compounds I as claimed in claim 1 for the preparation of compositions against animal pests or harmful fungi.

18. The use of the compounds I as claimed in claim 1 for controlling animal pests or harmful fungi.

## Revendications

1. Dérivés de l'acide phénylacétique répondant à la formule I 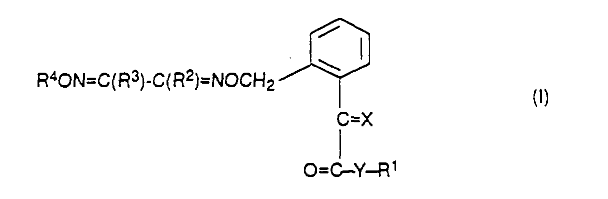 dans laquelle les symboles et l'indice ont les significations suivantes :
X NOCH₃, CHOCH₃ ou CHCH₃ ;
Y NR ou O :
R, R¹ l'hydrogène ou des groupes alkyle en C1-C4 ;
R² un groupe méthyle ;
R³ un groupe alcényle en C2-C6 ;
R⁴ l'hydrogène,
un groupe alkyle en C1-C6, alcényle en C2-C6, alcynyle en C2-C6, ces groupes pouvant être halogénés en totalité ou en partie et/ou pouvant porter un à trois des substituants suivants : cyano, hydroxy, amino, alcoxy en C1-C6, halogénoalcoxy en C1-C6, (alcoxy en C1-C6)carbonyle, alkylthio en C1-C6, alkylamino en C1-C6, di-(alkyle en C1-C6)amino, aminocarbonyle, (alkyle en C1-C6)aminocarbonyle, di-(alkyle en C1-C6)aminocarbonyle, alcényloxy, cycloalkyle en C3-C6, cycloalcoxy en C3-C6, les groupes cycliques pouvant eux-mêmes être halogénés en totalité ou en partie ou pouvant porter un à trois substituants alkyle en C1-C3 ou alcoxy en C1-C3 ;
et leurs sels.

2. Dérivés de l'acide phénylacétique de formule I selon revendication 1 dans laquelle R³ représente un groupe vinyle, allyle, prop-1-ényle, 2-méthyl-prop-1-ényle ou isopropényle.

3. Composés de formule I de la revendication 1, dans laquelle X représente NOCH₃, Y représente NH et R¹ un groupe méthyle.

4. Composés de formule I de la revendication 1, dans laquelle R¹ représente un groupe méthyle.

5. Un composé de formule

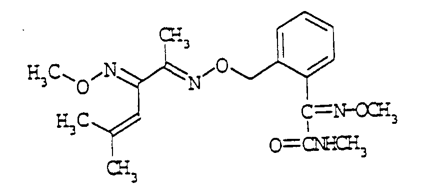

6. Un composé de formule

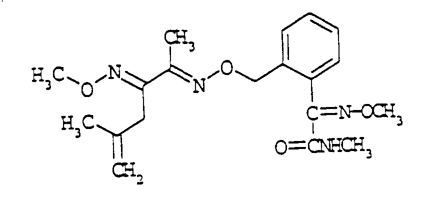

7. Un composé de formule

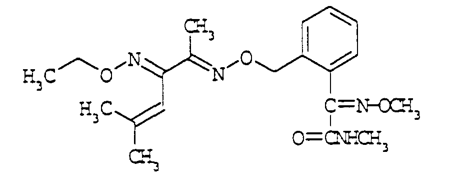

8. Procédé de préparation des composés de formule I de la revendication 1, **caractérisé en ce que** l'on fait réagir un dérivé benzylique de formule II 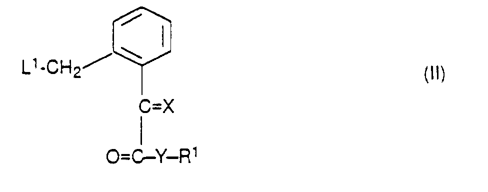 dans laquelle L¹ représente un groupe éliminable par échange nucléophile, de manière connue en soi, avec une hydroxyimine de formule III
R⁴ON=C(R³)-C(R²)=NOH (III)

9. Procédé de préparation des composés de formule I de la revendication 1, **caractérisé en ce que** l'on fait réagir un dérivé benzylique de formule II de la revendication 8, de manière connue en soi, avec une dihydroxyimine de formule IV
HON=C(R³)-C(R²)=NOH (IV)
la réaction donnant un composé de formule V 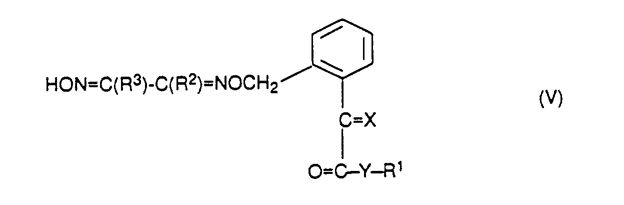 qu'on fait ensuite réagir avec un composé de formule VI
R⁴-L² (VI)
dans laquelle L² représente un groupe éliminable par échange nucléophile, la réaction donnant I.

10. Procédé de préparation des composés de formule I de la revendication 1, **caractérisé en ce que** l'on fait réagir un dérivé benzylique de formule II de la revendication 8, de manière connue en soi, avec un carbonylhydroxyimine de formule VII
O=C(R³)-C(R²)=NOH (VII)
la réaction donnant un composé de formule VIII 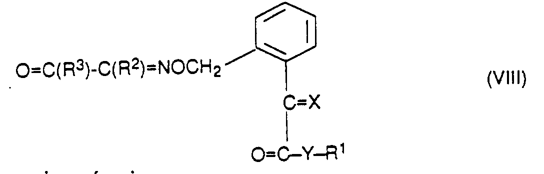 qu'on fait ensuite réagir
a) d'abord avec l'hydroxylamine ou l'un de ses sels puis avec un composé de formule VI (R⁴-L²) selon revendication 9, ou bien
b) avec une hydroxylamine ou un sel d'hydroxylammonium de formules respectives IXa et IXb 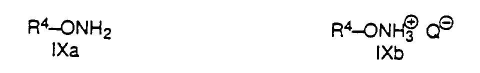 dans lesquelles Q^{⊖} représente l'anion d'un acide, ce qui donne I.

11. Composés de formule générale II selon revendication 8.

12. Composés de formule générale IV selon revendication 9.

13. Composés de formule générale V selon revendication 9.

14. Composés de formule générale VIII selon revendication 10.

15. Produit pour combattre les parasites animaux ou les mycètes nuisibles, contenant des additifs usuels et une quantité efficace d'un composé de formule I de la revendication 1.

16. Procédé pour combattre les parasites animaux ou les mycètes nuisibles, **caractérisé en ce que** l'on traite les parasites ou les mycètes nuisibles, leur habitat ou les végétaux, aires, matériaux ou locaux qu'on veut protéger contre eux par une quantité efficace d'un composé de formule I de la revendication 1.

17. Utilisation des composés I de la revendication 1, pour la préparation de produits pour la lutte contre les parasites animaux ou les mycètes nuisibles.

18. Utilisation des composés I de la revendication 1 pour la lutte contre les parasites animaux ou les mycètes nuisibles.
